(19) Europäisches Patentamt
European Patent Office
Office européen des brevets

(11) Veröffentlichungsnummer: **0 156 172**
**A2**

(12) **EUROPÄISCHE PATENTANMELDUNG**

(21) Anmeldenummer: **85102131.1**

(22) Anmeldetag: **27.02.85**

(51) Int. Cl.[4]: **C 07 D 317/72**
C 07 D 317/14, C 07 C 50/36
C 07 H 15/252

(30) Priorität: **09.03.84 FR 8403634**

(43) Veröffentlichungstag der Anmeldung:
**02.10.85 Patentblatt 85/40**

(84) Benannte Vertragsstaaten:
**AT BE CH DE GB IT LI LU NL SE**

(71) Anmelder: **LABORATOIRES HOECHST S.A.**
**Tour Roussel Nobel 3, Avenue du Général de Gaulle**
**F-92800 Puteaux(FR)**

(72) Erfinder: **Florent, Jean-Claude**
**23, rue de Causses**
**F-91940 Les Ulis(FR)**

(72) Erfinder: **Monneret, Claude**
**9, avenue Lamoricière**
**F-75012 Paris(FR)**

(74) Vertreter: **Reuter, Johann-Heinrich, Dr. et al,**
**HOECHST AKTIENGESELLSCHAFT Zentrale**
**Patentabteilung Postfach 80 03 20**
**D-6230 Frankfurt/Main 80(DE)**

(54) **Neue Anthracyclinone, Verfahren zu deren Herstellung, daraus enthaltene neue Glykoside (Anthracycline) und Verwendung dieser Glykoside als Arzneimittel.**

(57) Vorliegende Erfindung betrifft neue Anthracyclinone.
Diese Anthracyclinone stellen 3,13-O-Isopropyliden-anthra-cyclinone dar und entsprechen der nachfolgenden allgemeinen Formel:

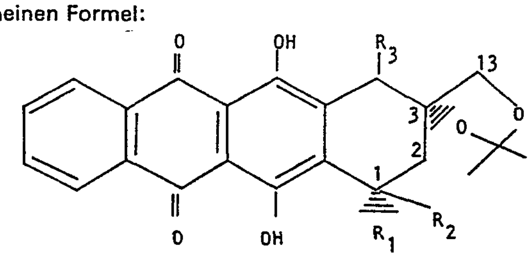

worin $R_1$, $R_2$ und $R_3$ für eine OH-Gruppe oder ein Wasserstoffatom stehen.
Anwendung dieser Anthracyclinone zur Herstellung von als Arzneimittel verwendbaren Anthracyclinen.

EP 0 156 172 A2

LABORATOIRES HOECHST S.A. HOE 84/S 002

Neue Anthracyclinone, Verfahren zu deren Herstellung, daraus erhaltene neue Glykoside (Anthracycline) und Verwendung dieser Glykoside als Arzneimittel

Vorliegende Erfindung betrifft neue Anthracyclinone, deren Herstellungsverfahren, daraus erhaltene neue Glykoside (Anthracycline) sowie die Verwendung dieser Glykoside als Arzneimittel.

Die Anthracyclinone oder 10-Desmethoxyanthracyclinone der unten angegebenen Formel I

I. 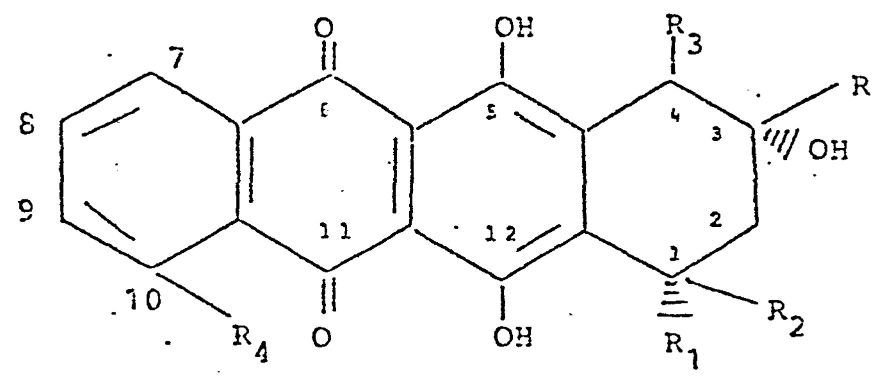

worin $R_1$, $R_2$ und $R_3$ gleich oder verschieden sein können und für eine OH-Gruppe oder ein Wasserstoffatom, R für eine Alkyl- oder Hydroxyalkylgruppe und $R_4$ für eine $OCH_3$-Gruppe oder ein Wasserstoffatom stehen, sind bekannt und werden weithin zur Bildung von hochwertigen Antibiotika und Antitumormitteln durch Glykosidierung verwendet (vgl. beispielsweise deutsches Patent 2 757 057 der Societa Farmaceutici Italia oder europäisches Patent 0 044 954 der Hoffmann-Laroche).

Betrachtung des oben dargestellten Moleküls zeigt sehr deutlich, dass man die vielfältigen, von diesem Molekül gebotenen Möglichkeiten noch gründlicher erforschen können und dabei zu pharmakologisch wahrscheinlich sehr interessanten Produkten kommen sollte. Leider werden die Möglichkeiten einer spezifischen Glykosidierung am OH in 1-Stellung durch die Funktionalisierung des Kohlenstoffatoms 3 durch eine hydroxyalkylierte Seitenkette erheblich eingeschränkt.

Es ist demnach die Aufgabe der vorliegenden Erfindung, eine neue Reihe Anthracyclinone zur Verfügung zu stellen, die es ermöglichen, unter einfachen und wirtschaftlichen Bedingungen eine Vielzahl von Glykosiden mit nahezu der Gewissheit zu synthetisieren, dass einige solche Derivate sicherlich einen hohen therapeutischen Wert aufweisen werden.

Gegenstand vorliegender Erfindung ist eine neue Reihe Anthracyclinone, die dadurch gekennzeichnet sind, dass sie 3,13-O-Isopropyliden-anthracyclinone darstellen und der nachfolgenden allgemeinen Formel II entsprechen:

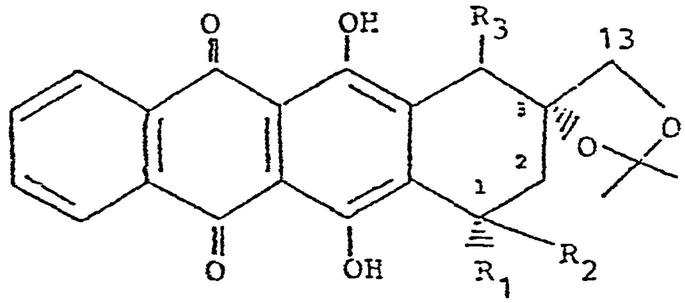

II.

worin $R_1$, $R_2$ und $R_3$ für eine OH-Gruppe oder ein Wasserstoffatom stehen.

Die Gegenwart dieses Isopropylidenacetals gestattet eine zweckmässige Orientierung des 1α- oder 1(S)-Hydroxyds und die Realisierung regiospezifischer Glykosidierungen, was für Anthracyclinone mit hydroxyalkylierten Seitenketten (in 3-Stellung) schwierig und manchmal unmöglich war.

Ein weiterer Gegenstand vorliegender Erfindung ist die Herstellung von 3,13-O-Isopropyliden-anthracyclinonen, welches durch die folgenden Stufen gekennzeichnet ist:

1) Umwandlung einer Saccharinsäure der nachfolgenden allgemeinen Formel III

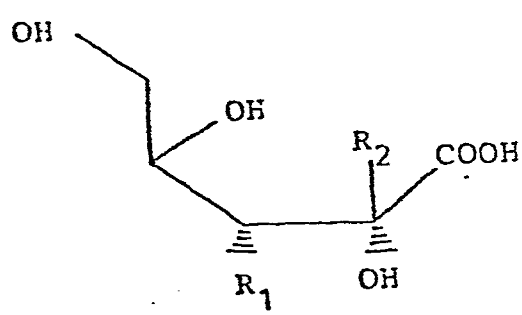

III.

worin $R_1$ = H oder OH und

0155172

$R_2 = CH_3$ oder $CH_2OH$

in das Lacton der allgemeinen Formel III.bis

III.bis

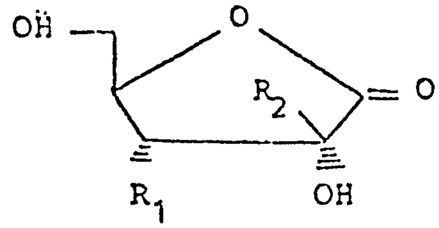

worin $R_1$ und $R_2$ die oben angegebenen Bedeutungen haben, dann Ueberführung des Lactons in einen Aldit und anschliessende Oxydation einer enständigen Alkoholfunktion zur Aldehydfunktion, wobei diese Umwandlung gemäss Formelbild IV erfolgt, wenn man von 3-Desoxy-2-hydroxymethyl-pentonsäure ausgeht, bzw. Formelbild V, wenn man von 2-Methyl-D-ribo-pentonsäure ausgeht.

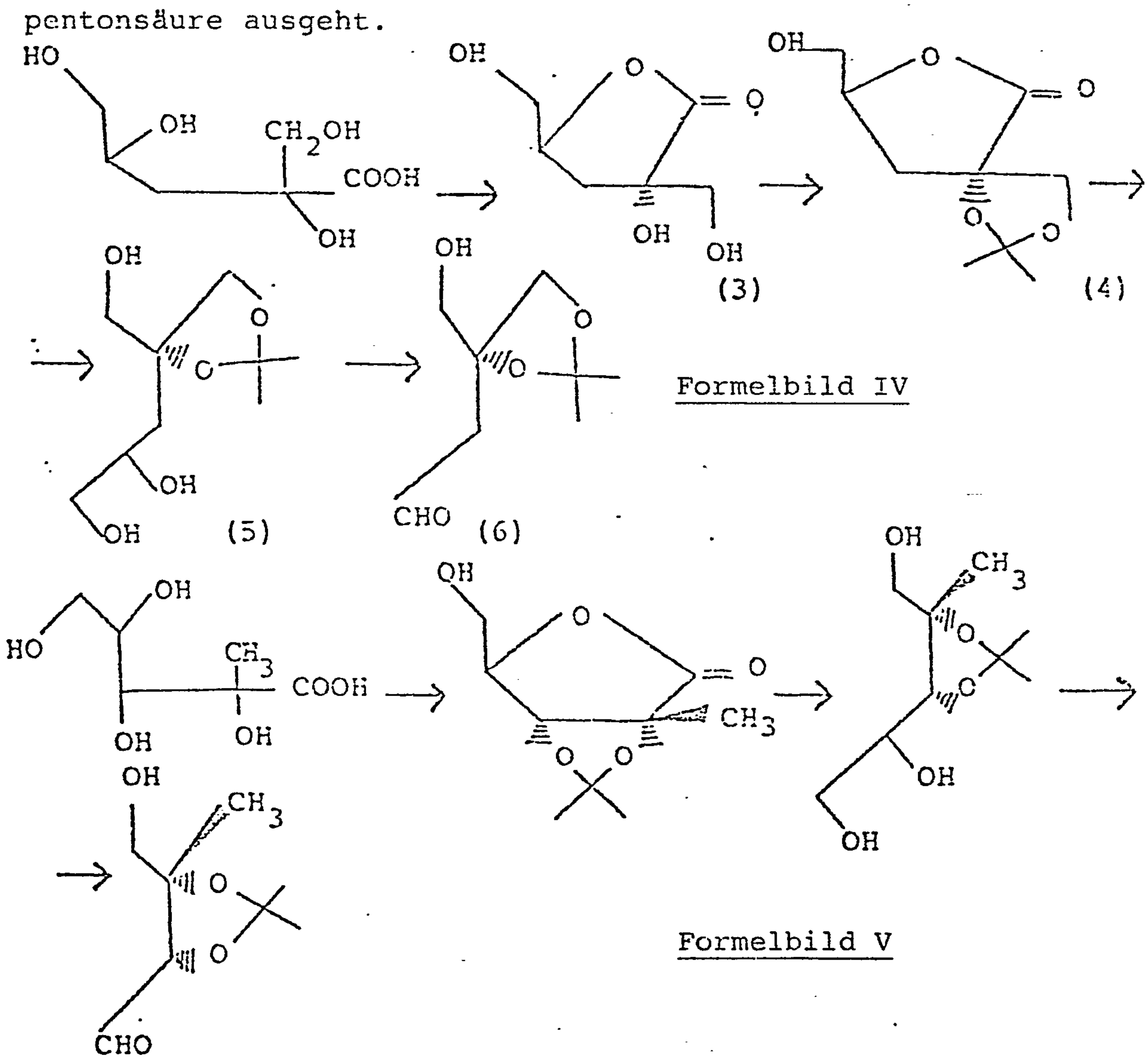

Formelbild IV

Formelbild V

2) Kondensationsreaktion des Aldehyds mit Leukochinizarin und nachfolgende Oxydation, Cyclisierung, Hydrogenolyse und schliesslich Hydrolyse gemäss nachfolgendem Formelbild VI:

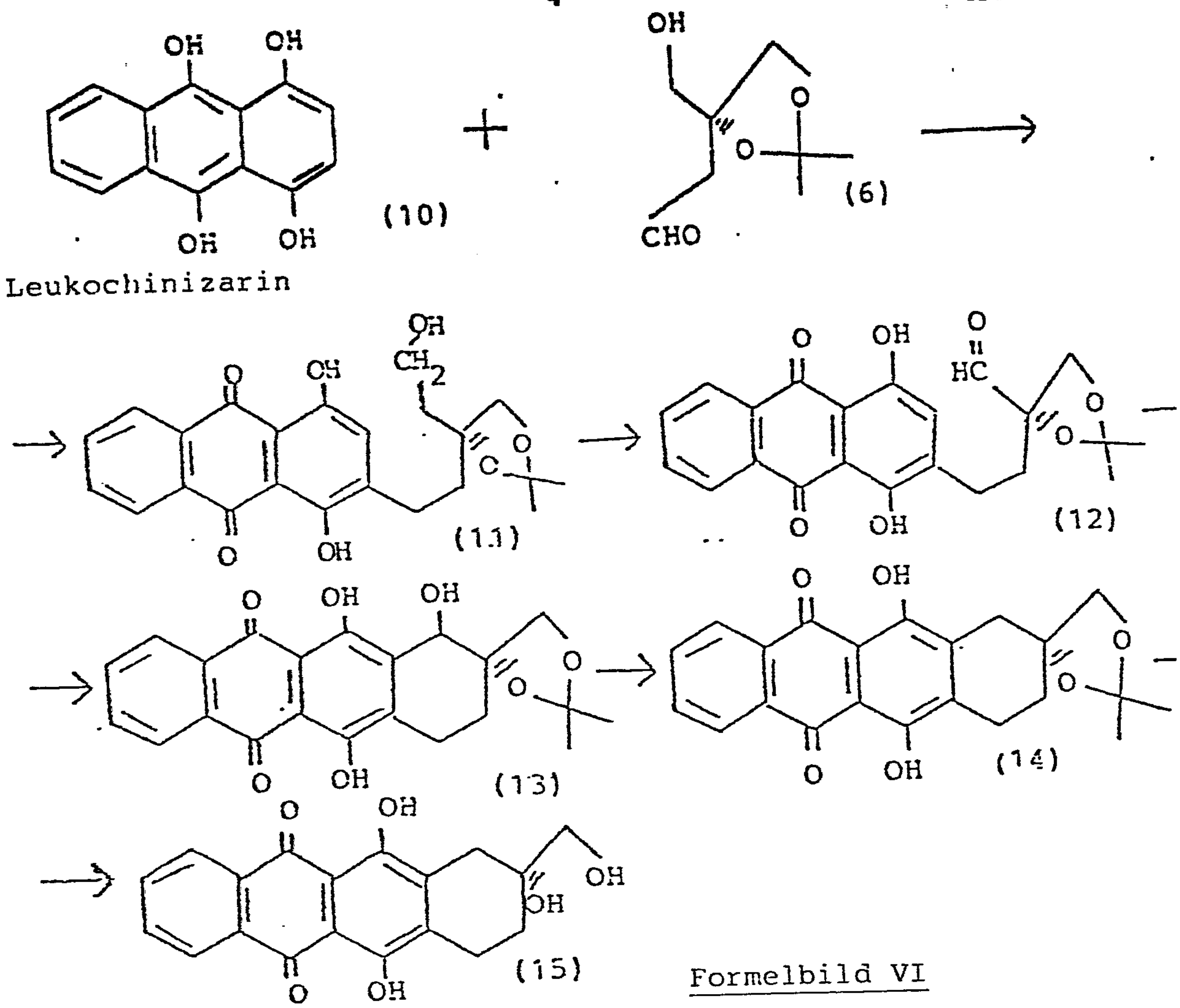

Leukochinizarin (10) (6)

(11) (12)

(13) (14)

(15)

Formelbild VI

Nach einer weiteren erfindungsgemässen Verfahrensvariante wandelt man das Isopropylidenlactonderivat in das entsprechende Amidderivat um, bildet davon ein Diisopropylidenderivat und überführt dieses dann in einen Aldit-aldeyhd, gemäss Formelbild VII:

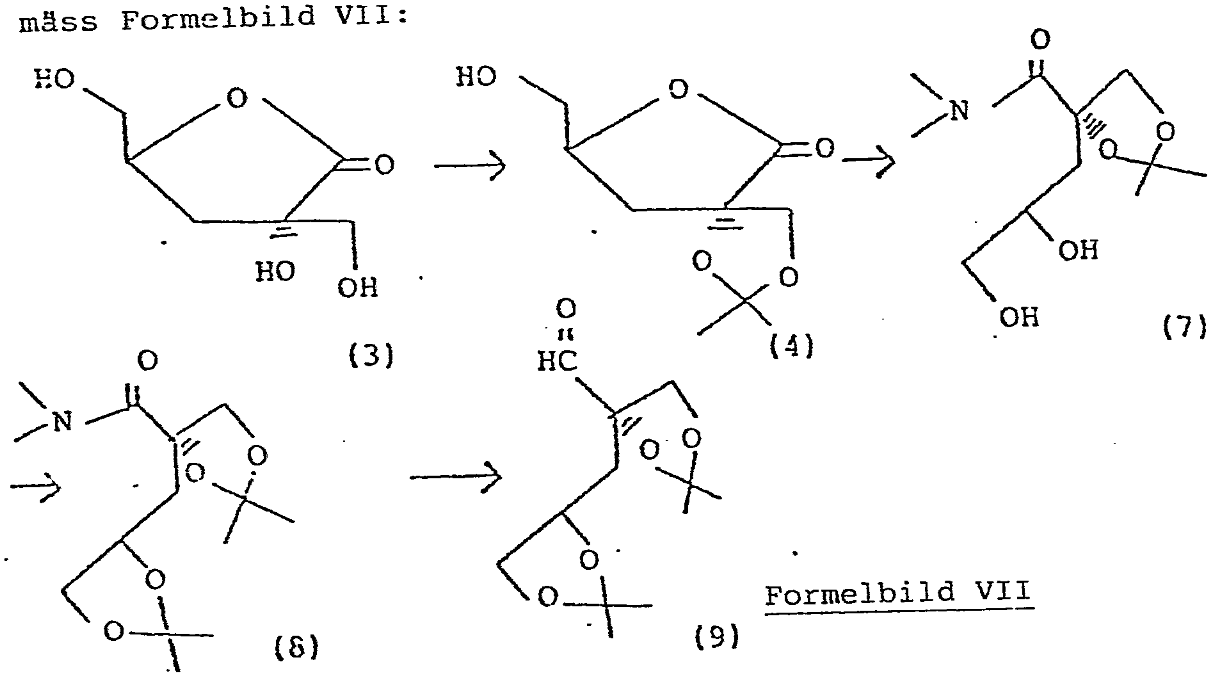

(3) (4) (7)

(8) (9)

Formelbild VII

Erfindungsgemäss wird der nach Formelbild VII erhaltene Aldehyd (9) gemäss nachfolgendem Formelbild VIII mit Leukochinizarin zum gewünschten cis-Anthracyclinonderivat kondensiert:

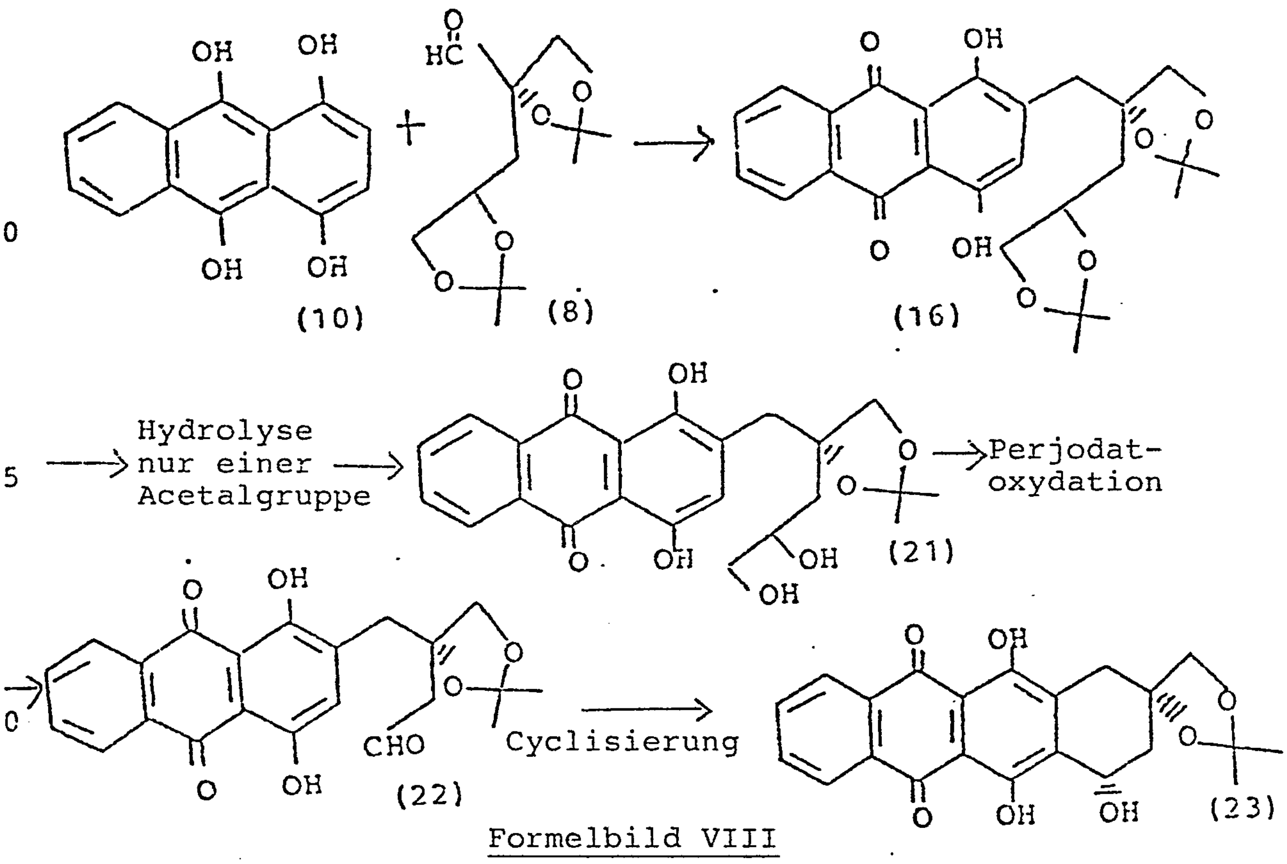


Formelbild VIII

Nach einer weiteren zweckmässigen Variante des erfindungsgemässen Verfahrens wird das Diacetal (16) zunächst hydrolysiert und dann oxydiert und anschliessend durch eine Aldolisierungsreaktion zu einem Gemisch aus cis- und trans-Anthracyclinonen cyclisiert, welches man durch Chromatographie trennt und gegebenenfalls durch Umsetzung mit α,α-Dimethoxypropan in saurem Medium in Isopropylidenderivate überführt, gemäss nachfolgendem Formelbild X:

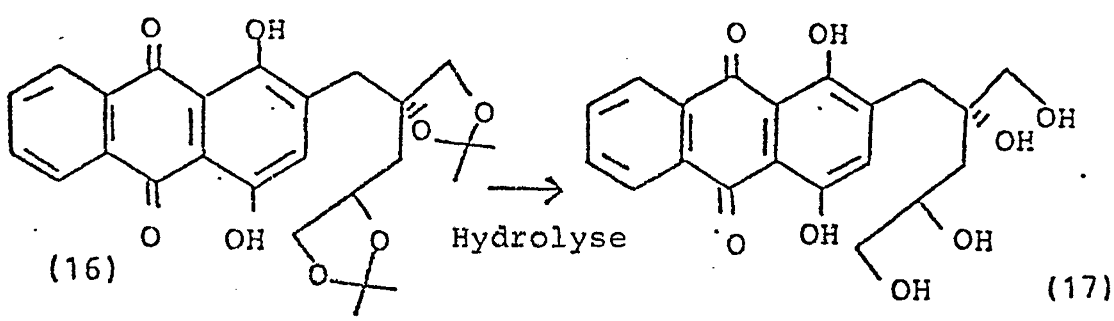

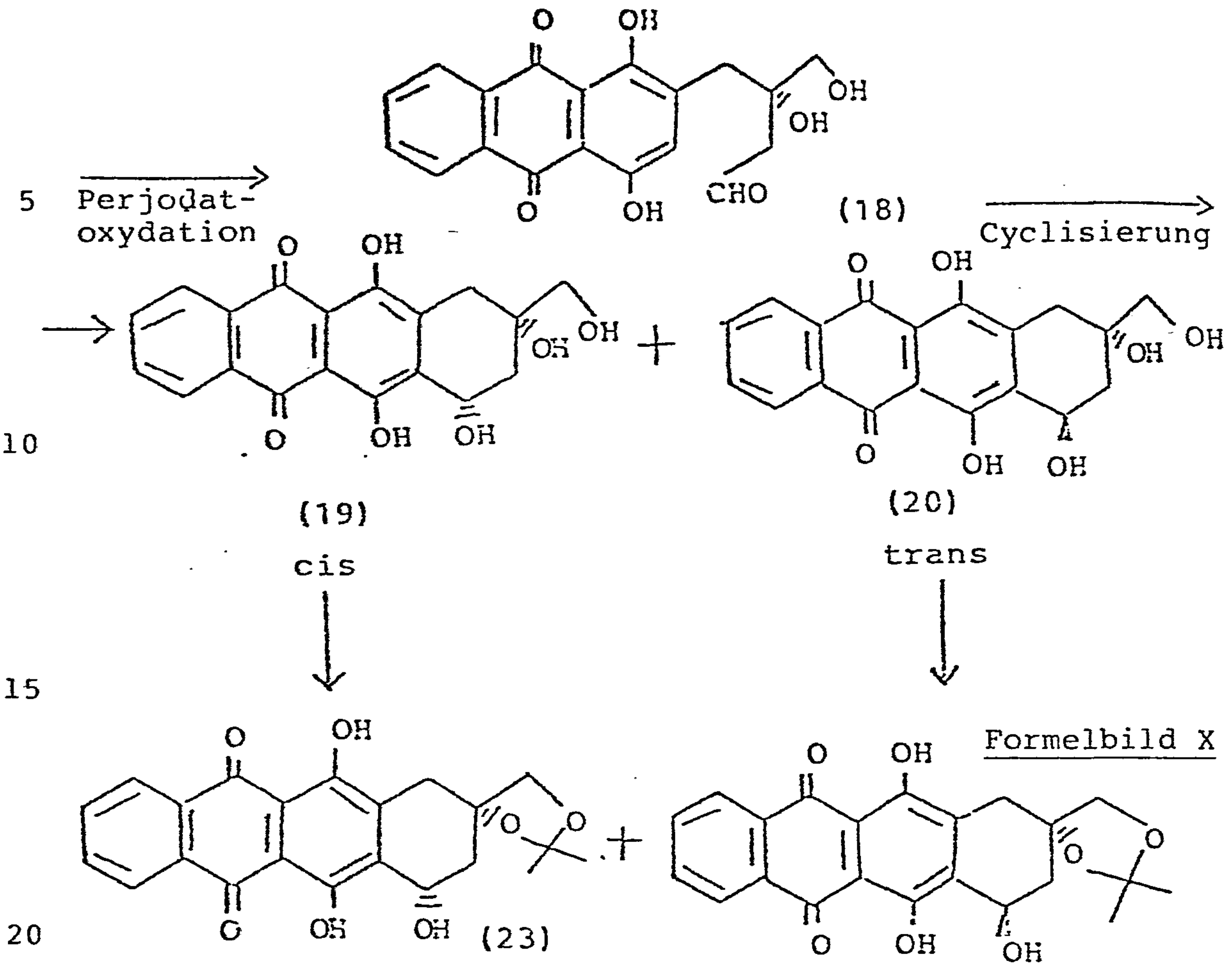


(18)

(19) cis

(20) trans

(23)

Formelbild X

Nach einer besonders vorteilhaften Ausbildung dieser Ausführungsform erfolgt die chromatographische Trennung der cis- und trans-Derivate nach Schutz der Alkoholfunktion der Hydroxymethylgruppe mit einer p-Methoxytriphenylmethylgruppe XI.

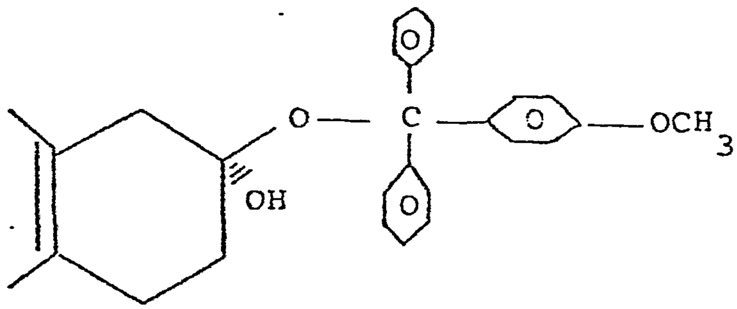

XI.

Gegenstand vorliegender Erfindung ist ferner die Umwandlung der Isopropylidenanthracyclinone in Anthracycline durch Glykosidierung mittels 3,4-Di-O-acetyl-2,6-didesoxy-α-L-lyxohexosechlorid (24) nach der Koenigs-Knorr-reaktion, gemäss nachfolgendem Formelbild XII:

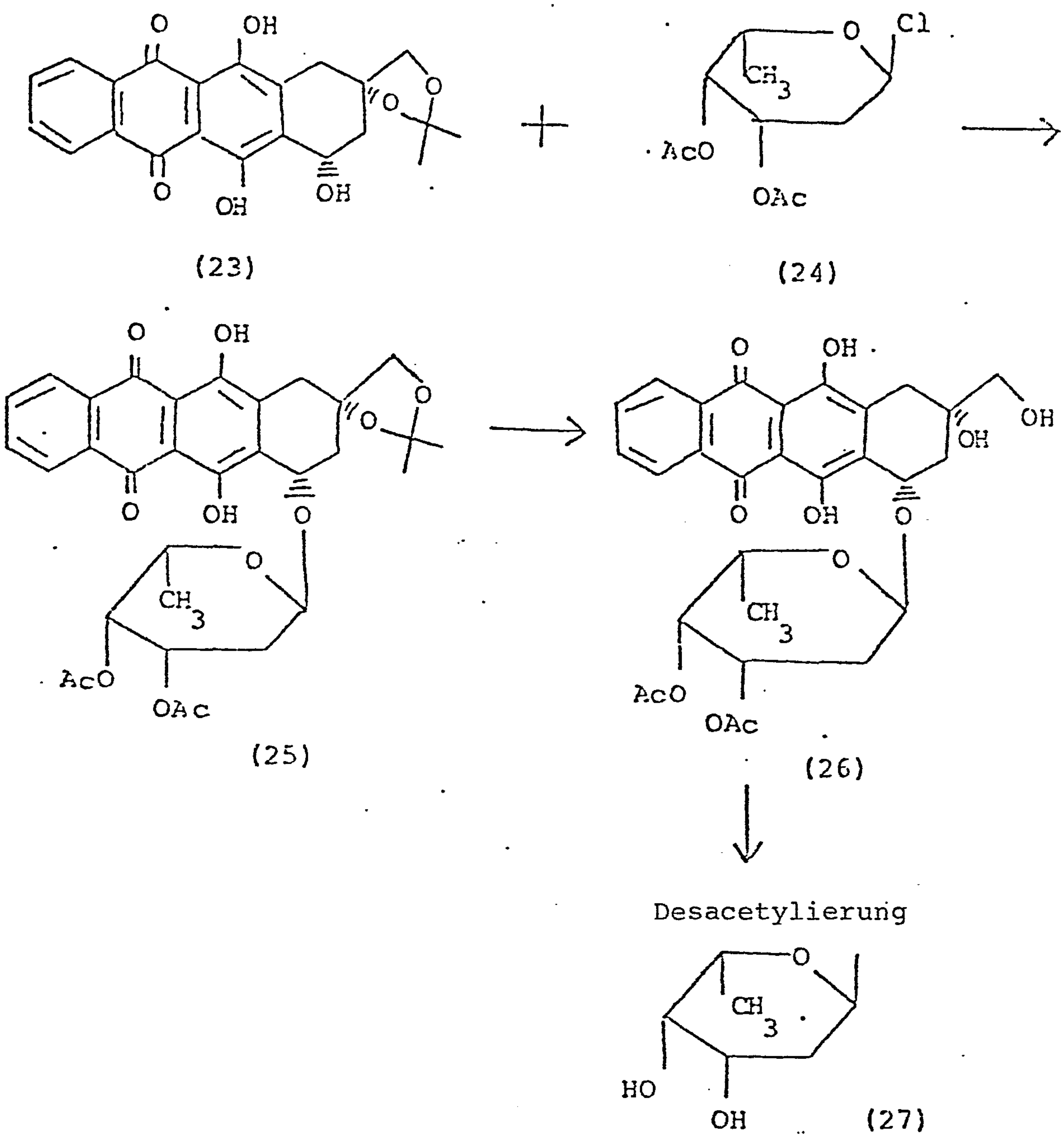


(23)

(24)

(25)

(26)

(27)

Neben den vorhergehenden Ausführungsformen umfasst die Erfindung ferner weitere, aus der nachfolgenden Beschreibung ersichtliche Ausführungsformen.

Gegenstand der Erfindung sind insbesondere die neuen Anthracyclinone, deren Herstellungsverfahren, die neuen Anthracycline und ihre Herstellungsverfahren sowie die Zusammensetzungen, insbesondere therapeutische Zusammensetzungen, in welche diese Derivate eingearbeitet werden.

Die Erfindung wird mit Hilfe der folgenden ergänzenden Beschreibung, die sich auf Ausführungsbeispiele der erfindungsgemässen Verfahren bezieht, noch besser verdeutlicht.

Es versteht sich jedoch, dass diese Ausführungsbeispiele einzig der Erläuterung des Erfindungsgegenstands dienen und diesen in keiner Weise einschränken.

BEISPIEL 1

α-D-1,4-Isosaccharino-lacton der Formel (3):

Dies wird nach den Angaben von Whistler und Bemiller (Methods in Carbohydr. Chem. 1963, Band II, S. 477-479) hergestellt.

BEISPIEL 2

2,2'-O-Isopropyliden-α-D-1,4-isosaccharino-lacton der Formel (4):

Dies wird nach Whistler und Bemiller (J. Org. Chem. 1961, Band XXVI, S. 2886) erhalten:
Schmelzpunkt 56°; $(\alpha)_D^{20}$ + 43° (c 1, Chloroform);
IR $\nu_{max.}^{Film}$: 1775, 1220 und 1060 $cm^{-1}$.

$^{13}C$-NMR ($CDCl_3$): 176,5 (C-1), 112,4 ($CMe_2$), 81,1 (C-4), 78,1 (C-2), 71,8 (C-2'), 63 (C-5), 35,7 (C-3), 26,4 und 25,7 (Isopropyliden-Me)

$^1H$-NMR ($CDCl_3$, 400MHz):
4,72 (1H, m, H-4)
4,34 (1H, d) und 4,12 (1H, d) (System AB, J = 4,5, $CH_2$-2')
3,97 (1H, dd) und 3,60 (1H, dd) (System ABX, J = 6 und J' = 1,5, $CH_2$-5)
3,03 (1H, OH)
2,45 (1H, m) und 2,34 (1H, m) (System ABX, J = 7 und J' = 3,5, H-3)
1,48 (6H, s, $CMe_2$)

Massenspektrum ($DCI/NH_3$): m/z 203 (M + 1, Spuren), 187

(M-15, 100%), 145 (12), 128 (12), 85 (19), 43 (36).

Analyse: $C_9H_{14}O_5$ (202,20).

| | | | |
|---|---|---|---|
| Berechnet % | C 53,46, | H 6,98, | O 39,56 |
| Gefunden | 53,54, | 7,02 | 39,57 |

BEISPIEL 3

3-Desoxy-2-C-hydroxymethyl-2,2'-O-isopropyliden-D-glycero-pentit der Formel (5):

a) Durch Reduktion mit Boran/Dimethylsulfidkomplex: man tropft bei Laboratoriumstemperatur eine 2m-Lösung (120 ml) des Boran/Dimethylsulfidkomplexes zu einer Lösung von 4 (10 g, 49,2 mMol) in wasserfreiem Tetrahydrofuran (250 ml). Nach 24 Stunden Rühren wird der Ueberschuss Reagenz durch vorsichtige Zugabe von Wasser unter Kühlung des Reaktionsgemischs im Eisbad zerstört. Das Wasser wird durch Vakuumdestillation entfernt und der Rückstand in Methanol aufgenommen. Dieses Methanol wird seinerseits bei vermindertem Druck abgedampft. Man wiederholt diesen Vorgang dreimal, bis man einen sirupösen Rückstand von 10 g Gewicht erhält.

b) Durch Reduktion mit $LiAlH_4$: zu einer Lösung des Lactons 4 (10 g, 49,2 mMol) in wasserfreiem Tetrahydrofuran (300 ml) gibt man in kleinen Portionen 4 g Lithiumaluminiumhydrid (während ungefähr 2 1/2 Stunden) bei $0^oC$. Das Reaktionsgemisch wird über Nacht bei Raumtemperatur gerührt, und der Hydridüberschuss wird dann in üblicher Weise zerstört (4 ml Wasser, 4 ml wässrige Natronlauge und schliesslich 12 ml Wasser). Man entfernt Unlösliches durch Filtrieren und dampft das Filtrat bei vermindertem Druck ein. Dabei erhält man 9,5 g sirupösen Rückstand, der laut Dünnschichtchromatographie (DSC) einheitlich ist.

BEISPIEL 4

3-C-Hydroxymethyl-3,3 -O-isopropyliden-D-glycerotetrose der Formel (6):

Man versetzt eine 21,6 g, d.h. 104,8 mMol, des in Beispiel 3 hergestellten Produkts in Methanol (200 ml) tropfenweise

0156172

mit in Wasser (200 ml) gelöstem Natriummetaperjodat (27 g, 106 mMol). Man rührt 1 Stunde bei Raumtemperatur und neutralisiert dann mit gesättigter wässriger Natriumbicarbonatlösung, entfernt Unlösliches durch Filtrieren und engt dann bei vermindertem Druck auf ein Volumen von ungefähr 200 ml (Entfernung des Methanols) ein und extrahiert die Suspension in üblicher Weise mit Essigester, was nach Abdampfen des Lösungsmittels bei vermindertem Druck einen Rückstand von 15,6 g Gewicht (86% Ausbeute) liefert, der laut DSC ($CH_2Cl_2$/MeOH, 95/5) einheitlich ist und bei niedriger Temperatur langsam kristallisiert: Schmelzpunkt <20°C; $(\alpha)_D^{20}$ + 18° (c 1, Aethanol, im Gleichgewicht);

IR $\nu_{max.}^{Film}$: 3410 (OH), 1730-1725 (CH=O) und 1375 $cm^{-1}$ ($CMe_2$).

NMR ($CDCl_3$, 400 MHz): 5,65 (1H, dd, J = 4, J' = 2) und 5,46 (1H, d, J = 4) (1H im ganzen, H-2); 4,10-3,78 (4H, m, $CH_2$ 4' und 5); 2,28-2,00 (2H, m, $CH_2$-3);

Massenspektrum (E.I.): m/z 159 ($M^{+\cdot}$ - 15, 100%), 143 (M - $CH_2OH$, 30%), 131 (M - $CH_2CHO$, 1,5%);

Analyse: $C_8H_{14}O_4$ (174,19):

| | | | |
|---|---|---|---|
| Berechnet % | C 55,16, | H 8,10, | O 36,74 |
| Gefunden: | C 55,32, | H 8,07, | O 36,70 |

## BEISPIEL 5

### 3-Desoxy-2-C-hydroxymethyl-2,2'-O-isopropyliden-N,N-dimethyl-D-ribonamid der Formel (7):

Man versetzt eine Lösung von 4 (50 g, 25 mMol) in Chloroform (150 ml) tropfenweise mit Dimethylaminlösung in Chloroform (100 ml in 500 ml $CHCl_3$) bei 0°C. Nach beendeter Zugabe lässt man das Reaktionsgemisch sich wieder auf Raumtemperatur erwärmen und dann 48 Stunden unter Rühren stehen. Das Verschwinden des Ausgangslactons wird durch DSC (Hexan/Aceton 1:1) verfolgt. Dann dampft man bei vermindertem Druck ein und reinigt den Rückstand durch Filtrieren über Kieselsäure (Hexan/Aceton 2:1 und danach 1:1). So gewinnt man 39,5 g reines 7 (Ausbeute 65%) als Harz: $(\alpha)_D^{20}$ -27° (c 1, Chloroform);

0156172

IR $\nu^{Film}_{max.}$: 1620 (Amid) und 1380 $cm^{-1}$ ($CMe_2$)

NMR ($CDCl_3$, 400 MHz):

4,68 (1H, d) und 3,98 (1H, d) (System AB, J = 9, $CH_2$-2').
3,77 (1H, m, H-4). 3,90 und 2,70 (2H, s, OH).
3,47 (1H, dd, J = 11,5 und J' = 6,5) und 3,57 (1H, dd, J = 11,5, J' = 3,5) (System ABX ($CH_2$-5).
3,27 (3H, s) und 2,95 (3H, s) ($NMe_2$)
2,00 (1H, dd, J = 14,5, J' = 10,5) und 1,89 (1H, dd, J = 14,5, J' = 2) (System ABX, $CH_2$-3).
1,42 (3H, s) und 1,34 (3H, s) ($CMe_2$).

BEISPIEL 6

3-Desoxy-2-C-hydroxymethyl-2,2':4,5-di-O-isopropyliden-N,N-dimethyl-D-ribonamid der Formel (8):

Zu einer Lösung von 7 (39 g, 158 mMol) in N,N-Dimethylformamid (500 ml) gibt man 130 ml α,α-Dimethoxypropan und dann Camphersulfonsäure (8 g, 34 mMol). Nach Rühren über Nacht bei Laboratoriumstemperatur giesst man das Reaktionsgemisch in mit Natriumbicarbonat gesättigtes Wasser (100 ml) und auf Eis. Nach 15 Minuten Rühren wird das Unlösliche abfiltriert und das Filtrat dann mit Aether/Essigester (1:1) extrahiert. Nach den üblichen Behandlungsschritten erhält man 41 g (90% Ausbeute), die laut DSC (Hexan/Aceton, 1:1) einheitlich sind, in Form eines Harzes, $(\alpha)^{20}_{D}$ -30° (c 1, Chloroform);
IR $\nu^{Film}_{max.}$: 1765 und 1630 (Amid) und 1375 $cm^{-1}$ ($CMe_2$).

NMR ($CDCl_3$, 400 MHz):

4,59 (1H, d) und 3,97 (1H, d) (System AB, J = 9, $CH_2$-2').
4,17 (1H, m, H-4).
4,08 (1H, dd, J = 8, J' = 5,5) und 3,48 (1H, dd, J = 8, J' = 7,5) (System ABX, $CH_2$-5)
3,27 (3H, s) und 2,96 (3H, s) ($NMe_2$).
2,25 (1H, dd, J = 13, J' = 7) und 1,95 (1H, dd, J = 13, J' = 5) (System ABX, $CH_2$-3)

1,45 (3H, s), 1,39 (3H, s), 1,35 (3H, s) und 1,34 (3H, s) (2 $CMe_2$).

Analyse: $C_{14}H_{25}NO_5$ (287,35)

Berechnet % C 58,51, H 8,77, N 4,87, O 27,84

Gefunden: C 58,65, H 8,92, N 4,77, O 27,80

BEISPIEL 7

3-Desoxy-2-C-hydroxymethyl-2,2':4,5-di-O-isopropyliden-D-ribose der Formel (9):

Zu einer Lithiumaluminiumhydridsuspension (1,32 g, 35 mMol) in wasserfreiem, auf -40°C gekühltem Aether (200 ml) gibt man das Ribonamid 8 (10 g, 35 mMol) in wasserfreiem Aether (100 ml) gelöst. Nach 4 Stunden Rühren bei -40°C beendet man die Reaktion durch vorsichtigen Zusatz, stets bei -40°C, von Essigester (ungefähr 50 ml) sowie nach Wiederansteigen der Temperatur auf 0°C durch Zusatz eines Wasser/Eisgemischs (≃ 50 ml) und schliesslich wässriger N-Natronlauge (50 ml). Die Suspension wird über eine Kieselgurschicht filtriert und das Filtrat mit Essigester extrahiert. Nach den üblichen Behandlungsschritten und Abdampfen des Lösungsmittels bei vermindertem Druck erhält man 7 g (78% Ausbeute) 9, das für die nachfolgende Stufe genügend rein ist. Eine Analysenprobe von 9 erhält man durch Säulenchromatographie über Kieselsäure (Hexan/Essigester 2:1): Harz; $(\alpha)_D^{20}$ -53° (c 1, Chloroform);

IR $\nu_{max.}^{Film}$: 2840 und 1730 (Aldehyd) und 1375 $cm^{-1}$ ($CMe^2$).

NMR ($CDCl_3$, 400 MHz):

4,31 (1H, m, H-4)

4,23 (1H, d) und 3,83 (1H, d) (System AB, J = 9, $CH_2$-2')

4,12 (1H, dd) und 3,59 (1H, dd) (System ABX, J = 8, J' = 6, $CH_2$-5).

2,15 (1H, dd, J = 13,5, J' = 9) und 1,84 (1H, dd, J' = 4) (System ABX, $CH_2$-3)

1,46 (3H, s), 1,45 (3H, s), 1,35 (3H, s) und 1,32 (3H, s) (2 $CMe_2$)

Analyse, berechnet für $C_{12}H_{20}O_5$.1/2 $H_2O$) (253,28):

C 56,91, H 8,36, O 34,72
Gefunden: C 57,00, H 8,35, O 34,84

BEISPIEL 8

1,4-Dihydroxy-2-(3-3(R)-C-hydroxymethyl-3,3'-O-isopropyliden-pentan-3,4-diol)-anthrachinon der Formel (11):

Unter Argon bei 0°C gibt man tropfenweise Essigsäure (36,5 ml) zu einem Gemisch aus Isopropylalkohol (150 ml) und Piperidin (120 ml) und versetzt dann nacheinander mit 6, dem Produkt aus Beispiel 4 (7 g, 40,2 mMol), gelöst in Isopropylalkohol (40 ml), und Leukochinizarin, ebenfalls in Isopropanollösung (15,5 g, 64,25 mMol in 50 ml). Das Reaktionsgemisch wird über Nacht unter Argon zum Rückfluss erhitzt und dann nach Abkühlen und Durchperlen von Luft (30 Minuten bis 1 Stunde) mit wässriger 1n-HCl angesäuert. Der sich bildende Niederschlag wird durch Filtrieren abgetrennt und mit Wasser gewaschen, und das Filtrat wird dann in üblicher Weise mit Dichlormethan extrahiert. Dann vereinigt man den Niederschlag mit dem Extraktionsrückstand, was insgesamt 15 g Rohprodukt ergibt, das man über Kieselsäure filtriert (Hexan/Dichlormethan, 1:1). Dabei gewinnt man 12 g des gewünschten Produkts (75% Ausbeute), das laut DSC (Dichlormethan) einheitlich ist. Zur Analyse wird eine Probe umkristallisiert (Aceton).
Schmelzpunkt 203-205°, $(\alpha)_D^{20}$ +26° (c 0,1, Chloroform);
IR $\nu_{max.}^{Nujol}$: 3460 (OH), 1625 und 1585 $cm^{-1}$ (Chinon mit Wasserstoffbrücken)

NMR ($CDCl_3$, 400 MHz):
8,26 (2H, m) und 7,76 (2H, m) (System AA' und BB', 4H aromatisch)
7,10 (1H, s, H-2)
3,87 (1H, d) und 3,76 (1H, d) (System AB, J = 9, $CH_2$-3')
3,67 (1H, d) und 3,61 (1H, d) (System AB, J = 10, $CH_2OH$).
2,79 (2H, m, Benzyl-$CH_2$)
2,16 (1H, s, OH)

1,96 (2H, t, J = J' = 8,5, $CH_2$-2).
1,49 (3H, s) und 1,46 (3H, s) ($CMe_2$).

Massenspektrum: (E.I.) m/z 398 ($M^+$, 17%), 383 (M - 15, 10%), 340 (M - Aceton, 5%) und 309 (M - 89, 100).

Analyse, berechnet für $C_{22}H_{22}O_7$ (398,40):
C 66,32, H 5,57, O 28,11
Gefunden: C 66,43, H 5,63, O 28,22

BEISPIEL 9

1,4-Dihydroxy-2-(3-3(R)-hydroxy-3-C-hydroxymethyl-3,3'-O-isopropyliden-pentanal-4)-anthrachinon der Formel (12):

a) Oxydation nach Corey u.a.: Man versetzt eine Lösung von N-Chlorsuccinimid (1,5 g, 11,27 mMol) in wasserfreiem Toluol (25 ml) mit Dimethylsulfid (1,1 ml, 14,96 mMol). Das Reaktionsgemisch, in welchem ein weisser Niederschlag auftritt, wird auf -25°C abgekühlt und dann tropfenweise unter ständigem Rühren mit einer Lösung des Produkts aus Beispiel 8 (1 g, 2,51 mMol) in Toluol/THF-Gemisch (50 ml, 1:1) versetzt. Nach beendeter Zugabe hält man die Temperatur 2 Stunden bei -25°C und gibt dann Triäthylamin (0,5 ml in 10 ml Toluol) dazu. Man lässt das Reaktionsgemisch sich auf Raumtemperatur erwärmen und rührt noch weitere 15 Stunden. Dann verdünnt man mit Wasser und extrahiert mit Aether. Die ätherische Phase wird mit wässriger 1%iger HCl gewaschen und in üblicher Weise weiterbehandelt. Durch Filtrieren über Kieselsäure lässt sich 0,55 g reines kristallisiertes Produkt erhalten: Schmelzpunkt 94-98° (Hexan/Aceton); $(\alpha)_D^{20}$ +8° (c 0,1, Aceton) und +17° (c 0,1, Dioxan); IR $\nu_{max.}^{Nujol}$: 1730 (CHO), 1625 und 1585 $cm^{-1}$ (Chinon mit Wasserstoffbrücken)

NMR ($CDCl_3$, 400 MHz):
8,22 (2H, m) und 7,75 (2H, m) (System AA' und BB', 4H aromatisch)
7,05 (1H, s, CH=O)
4,23 (1H, d) und 3,90 (1H, d) (System AB, $CH_2$-3')
2,87 (1H, m) und 2,65 (1H, m) (System AB, Benzyl-$CH_2$).

2,15 (1H, m) und 1,98 (1H, m) (System AB, $CH_2$-2). 1,76 (3H, s) und 1,46 (3H, s) ($CMe_2$).

Massenspektrum DCI/$NH_3$): m/z 414 (M + 1 + $NH_3$, 10%), 397 (M + 1, 100), 381 (20), 309 (10) und 110 (5).

Analyse, berechnet für $C_{22}H_{20}O_7$ (396,38):

% C 66,66, H 5,09, O 28,25

Gefunden: C 66,43, H 5,20, O 28,17

b) Oxydation nach Pfitzner-Moffatt: Zu einer Lösung des Produkts aus Beispiel 8 (1 g, 2,51 mMol) in einem Gemisch aus wasserfreiem Toluol (100 ml) und wasserfreiem Dimethylsulfoxyd (30 ml) gibt man Pyridin (0,4 ml, 4,9 mMol), Trifluoressigsäure (0,1 ml, 1,38 mMol) und Dicyclohexylcarbodiimid (1,83 g, 8,88 mMol). Man rührt 4 Stunden bei Raumtemperatur, verdünnt dann mit Wasser und extrahiert mit Aether. Dabei verbleibt ein roher Rückstand (0,95 g), der dann wie zuvor über Kieselsäure filtriert wird. So erhält man 0,80 g reines kristallisiertes Produkt (80% Ausbeute) mit denselben Kenndaten wie für das gemäss a) erhaltene Produkt beschrieben.

BEISPIEL 10

1,2,3,4,6,11-Hexahydro-3(R),4(R und S),5,12-tetrahydroxy-3-C-hydroxymethyl-3,13-O-isopropyliden-6,11-naphthacendion der Formel (13):

Man gibt bei 0°C unter Argon eine wässrige, Kaliumhydroxyd (365 mg) und Natriumhydrosulfit (460 mg) enthaltende Lösung (25 ml) zu einer Lösung von 12 (1,15 g, 2,9 mMol) in Methanol/THF-Gemisch (200 ml, 1:1). Nach 3 Stunden Rühren bei 0°C unter Argon oxydiert man das Gemisch, indem man ungefähr 30 Minuten lang Luft durchperlt. Nach Ansäuern mit wässriger 1n-HCl (10 ml) extrahiert man in üblicher Weise, wobei man einen kristallinen Rückstand von 1,10 g Gewicht gewinnt. Dieser wird über Kieselsäure chromatographiert (Dichlormethan) und liefert 690 mg Produkt (60% Ausbeute), das laut DSC mit Dichlormethan als Laufmittel einheitlich ist, aber bei DSC in Hexan/Essigester (3:1) zeigt sich die Gegenwart zweier Verbindungen (die beiden C-4-Isomeren).

Die Natur des Gemischs wird durch das NMR-Spektrum bestätigt.

NMR (Pyridin-$d_5$, 270 MHz):

13,78 (2H, schlecht aufgelöster Rücken, OH-Wasserstoffbrücken)

8,35 (2H, m) und 7,73 (2H, m) (System AA' und BB', 4H aromatisch)

5,40 (s) und 5,36 (s) (1H insgesamt, H-4)

4,74 (d), 4,17 (d) }
4,07 (d) und 3,87 (d) } (2 Systeme AB, $CH_2$-13)

3,25-3,03 und 2,94-2,68 (2H insgesamt, m, $CH_2$-1)

2,09-1,87 (2H, m, $CH_2$-2)

Massenspektrum (DCI/$NH_3$): m/z 397 (M + 1, 100%), 339 (M + 1 - Aceton, 8), 321 (M + 1 - Acetone - 18, 22), 225 (27).

Analyse: $C_{22}H_{20}O_7$ (396,38).

| | | | |
|---|---|---|---|
| Berechnet %: | C 66,66, | H 5,09, | O 28,25 |
| Gefunden: | C 66,55, | H 5,12, | O 28,37 |

BEISPIEL 11

1,2,3,4,6,11-Hexahydro-3(R),5,12-trihydroxy-3-C-hydroxymethyl-3,13-O-isopropyliden-6,11-naphthacendion der Formel (14):

Man rührt eine äthanolische Lösung (10 ml) des Produkts aus Beispiel 10 (30 mg) 1 Stunde lang in einer Wasserstoffatmosphäre in Gegenwart von 10%igem Palladium auf Bariumsulfat (20 mg). Der Katalysator wird dann durch Filtrieren entfernt und das Filtrat mittels Durchperlen von Luft für 10 Minuten wiederoxydiert. Man verdampft das Lösungsmittel bei vermindertem Druck und kristallisiert den Rückstand aus Aceton. Dabei erhält man 20 mg reines Produkt: Schmelzpunkt 231-232°C; $(\alpha)_D^{20}$ - 52° (c 0,03, Chloroform);
IR $\nu_{max.}^{CHCl_3}$: 1625 und 1590 (Chinon mit Wasserstoffbrücken) und 1380 $cm^{-1}$ ($CMe_2$).

NMR ($CDCl_3$, 270 MHz):

13,42 (s) und 13,40 (s) (2 OH-Wasserstoffbrücken)

8,32 (2H, m) und 7,81 (2H, m) (System AA' und BB', 4H aromatisch)

3,89 (2H, s, $CH_2$-13)

3,05-2,81 (4H, m, $CH_2$-4)

2,17-2,04 (1H, m) und 1,86-1.70 (1H, m) ($CH_2$-2)

1,43 (3H, s) und 1,42 (3H, s) ($CMe_2$)

Massenspektrum (DCI/$NH_3$): m/z 398 (M + 1 + $NH_3$, 5%), 381 (M + 1, 100), 323 (M + 1 - 58, 10).

Analyse: $C_{22}H_{20}O_6$ (380,38):

Berechnet %: C 69,46, H 5,30, O 25,24

Gefunden: C 69,53, H 5.27, O 25,17

BEISPIEL 12

1,2,3,4,6,11-Hexahydro-3(R),5,12-trihydroxy-3-C-hydroxymethyl-6,11-naphthacendion der Formel (15):

Man versetzt eine Lösung des Produkts aus Beispiel 11 (9 mg) in Methanol (2 ml) mit wässriger 1n-HCl (0,4 ml). Man rührt 4 Stunden lang und neutralisiert dann mit Amberlit-Harz IR 45 ($OH^-$). Nach Verdampfung des Lösungsmittels bei vermindertem Druck erhält man 8 mg reines kristallisiertes 15: Schmelzpunkt 235-238°; $(\alpha)_D^{20}$ -32° (c 0,062, Dioxan); IR $\nu_{max.}^{CHCl_3}$: 3250 (OH), 1625 und 1590 $cm^{-1}$ (Chinon mit Wasserstoffbrücken)

NMR (Pyridin-$d_5$, 400 MHz)

8,39 (2H, m) und 7,75 (2H, m) (System AA' und BB', 4H aromatisch)

4,09 (2H, s, System $A_2$, $CH_2$-13)

3,44 (1H, d) und 3,21 (1H, d) (System AB, J = 19, $CH_2$-1)

3,39-3,11 (4H, m, $CH_2$-4 und 2 OH)

2,30 (1H, m) und 2,08 (1H, m) ($CH_2$-2)

Massenspektrum (E.I.): m/z 340 ($M^{+\cdot}$, 100%), 308 (M - 31, 88) und 291 (M - 31 - 18, 68). Molekülzacke bei hoher Auflösung:

Berechnet für $C_{19}H_{16}O_6$ = 340,336;

Gefunden: 340,0944.

BEISPIEL 13

1,4-Dihydroxy-2-(3'-desoxy-2'-C-hydroxymethyl-2',2":4',5'-di-O-isopropyliden-D-ribityl)-anthrachinon der Formel (16):

Zu einer Lösung von Piperidin (90 ml, 900 mMol) in 170 ml Isopropylalkohol gibt man Eisessig (59,8 ml, 1,02 Mol). Unter ständiger Kühlung auf $0^{o}C$ versetzt man dann mit dem Aldehyd aus Beispiel 7 (13,7 g, 56 mMol), gelöst in 170 ml Isopropylalkohol, und dann mit Leukochinizarin (13,57 g, 56 mMol), ebenfalls in 170 ml Isopropylalkohol gelöst. Man erhitzt das Reaktionsgemisch 20 Stunden lang unter Argon zum Rückfluss. Nach Abkühlung perlt man zur Oxydation ungefähr 1 Stunde lang Luft hindurch und giesst auf HCl-Eiswasser (35 ml 12n-HCl in 75 ml Wasser + 75 g Eis). Der gebildete Niederschlag wird abfiltriert und das Filtrat mit Dichlormethan in üblicher Weise extrahiert. Der so erhaltene Rückstand wird mit dem Niederschlag vereinigt, was insgesamt 26 g Rohprodukt ergibt. Filtrieren über Kieselsäure (Hexan/Dichlormethan 2:1) liefert 19,2 g (75% Ausbeute) reines Produkt. Eine Probe wird zur Analyse aus Hexan/Aceton (1:2) umkristallisiert: Schmelzpunkt $130^{o}C$;
$(\alpha)_D^{20}$ $-70^{o}$ (c 0,15, Dioxan);
IR $\nu_{max.}^{Nujol}$: 1640 und 1600 $cm^{-1}$ (Chinon mit Wasserstoffbrükken) und 1385 $cm^{-1}$ ($CMe_2$).

NMR ($CDCl_3$, 400 MHz):
13,58 (1H, s) und 12,88 (1H, s) (2 phenolische OH mit Wasserstoffbrücken).
8,25 (2H, m) und 7,84 (2H, m) (System AA' und BB', 4H aromatisch)
7,48 (1H, s, H-3)
4,43 (1H, m, J = J' = 7,5; J" = J"' = 5,5, H-4')
4,1? (1H, dd, J = 8, J' = 5,5) und 3,35 (1H, dd, J = 8, J' = 7,5) (System ABX, $CH_2$-5')
3,97 (1H, d) und 3,95 (1H, d) (System AB, J = 9,5, $CH_2$-2")
3,26 (1H, d) und 3,11 (1H, d) (System AB, J = 14, $CH_2$-1')
1,90 (2H, m, $CH_2$-3')

1,44 (3H, s), 1,40 (6H, s) und 1,26 (3H, s) (2 CMe$_2$).

Massenspektrum (E.I.): m/z 469 ($M^{+\cdot}$ + 1, Spuren); 454 ($M^{+}$ + 1 - 15, Spuren); 411 (M - Aceton, Spuren); 395 (M - Aceton - 15, 15%); 254 (13%); 215 (35%); 101 (100%)

Analyse: Berechnet für $C_{26}H_{25}O_8$ (468,48) %:
C 66,65, H 6,02, O 27,32

Gefunden: C 66,75, H 6,00, O 27,43

## BEISPIEL 14

**1,4-Dihydroxy-2-(1',3'-Didesoxy-2'-C-hydroxymethyl-D-ribityl)-anthrachinon der Formel (17):**

Zu einer Lösung des vorangehenden Produkts 16 (14,5 g, 30 mMol) in einem Gemisch aus Tetrahydrofuran und Methanol (500 ml, 1:1) gibt man wässrige n-Salzsäure (500 ml). Man erhitzt 3 Stunden auf 70$^{o}$ und engt das Reaktionsgemisch dann bei vermindertem Druck ein, um das THF und Methanol zu entfernen. Dabei beobachtet man einen Niederschlag, der bei Zusatz von Eis und Wasser zunimmt. Der Niederschlag wird abfiltriert und dann getrocknet. Dies ergibt 12 g des gewünschten Produkts (quantitative Ausbeute), und zur Analyse wird eine Probe umkristallisiert (Methanol/Aether): Schmelzpunkt 90-92$^{o}$; $(\alpha)_D^{20}$ +12,5$^{o}$ (c 0,08, Dioxan);

IR $\nu_{max.}^{Nujol}$: 3350 (OH), 1640 und 1600 $cm^{-1}$ (Chinon mit Wasserstoffbrücken).

NMR ($CD_3OD$, 400 MHz):

8,24 (2H, m) und 7,87 (2H, m) (System AA' und BB', 4H aromatisch)

7,38 (1H, s, H-3)

3,57 (1H, d) und 3,55 (1H, d) (System AB, J = 12, $CH_2$-2")

3,41 (2H, m, $CH_2$-5')

3,17 (1H, d) und 2,81 (1H, d) (System AB, J = 13,5, $CH_2$-1')

1,80 (1H, dd, J = 15, J' = 2) und 1,53 (1H, dd, J = 15 und J' = 10) (System ABX, $CH_2$-3')

4,02 (1H, m, H-4')

Massenspektrum (E.I.): m/z 388 ($M^{+\cdot}$, Spuren), 370 (M - 18, Spuren), 321 (7) und 257 (100).

Analyse: Berechnet für $C_{20}H_{20}O_8$ (388,36):
C 61,85, H 5,19, O 32,96
Gefunden: C 62,02, H 5,17, O 32,80

BEISPIEL 15

1,4-Dihydroxy-2-(2'(S)-hydroxy-2'-C-hydroxymethyl-butanal-4')-anthrachinon der Formel (18):

Eine Lösung des Produkts aus Beispiel 14 (2,46 g, 6,18 mMol) in THF/Methanol (200 ml, 1:1) versetzt man mit wässriger Natriumperjodatlösung (1,4 g in 15 ml Wasser, d.h. 6.5 mMol) und dann mit Eisessig (8 ml). Nach 3 Stunden Rühren bei Raumtemperatur filtriert man das Reaktionsgemisch und extrahiert das Filtrat nach Verdünnen mit 200 ml Wasser mehrmals mit Essigester. Der nach Abdampfen des Lösungsmittels bei vermindertem Druck erhaltene Rückstand wird in Toluol (2 x 50 ml) aufgenommen und erneut eingedampft. Dabei erhält man schliesslich einen kristallinen Rückstand vom Gewicht 2.16 g (98%), der laut DSC ($CH_2Cl_2$/Methanol, 95:5) einheitlich ist. Eine Probe wird zur Analyse umkristallisiert (Hexan/Aceton): Schmelzpunkt 95-100°C, $(\alpha)_D^{20}$ + 78° (c 0,048, Dioxan);

IR $\nu_{max.}^{Nujol}$: 3400 (OH), 1640 und 1600 $cm^{-1}$ (Chinon mit Wasserstoffbrücken);

NMR ($CDCl_3$, 400 MHz) am Gemisch der α- und β-Anomeren:
8,33 (2H, m) und 7,85 (2H, m) (System AA' und BB', 4H aromatisch)
5,73 (dd, J = 5, J' = 3,5) und 5,53 (dd, J = 5, J' = 1) H-4').
4,18-3,93 (2H, d, System AB, $CH_2$-2").
3,23 (s) und 3,14 (s) (System $A_2$, $CH_2$-1').
2,16 (m) und 2,05 (m) $CH_2$-3').
1,69 (1H, s, OH) und 3,78 (1H, d, OH).

Massenspektrum (E.I.): m/z 356 ($M^{+\cdot}$, 5%), 338 (M - 18, 4), 320 (M - 18 - 18, 50), 312 (M - $CH_2CHO$, 30) und 253 (100).

Analyse: Berechnet für $C_{19}H_{16}O_7$ (356,32):

C 64,04, H 4,53, O 31,43

Gefunden: C 64,25, H 4,50, O 31,25

BEISPIEL 16

1,2,3,4,6,11-Hexahydro-3(R),5,12-trihydroxy-3-C-hydroxymethyl-6,11-naphthacendion der Formel (15):

Zu einer Lösung des Produkts aus Beispiel 15 (2 g, 5,6 mMol) in THF/Methanol (200 ml, 1:1) gibt man unter Argon eine wässrige, Natriumhydroxyd (0,8 g) und Natriumhydrosulfit (1,5 g, d.h. 8.63 mMol) enthaltende Lösung (10 ml). Das Reaktionsgemisch lässt man bei Raumtemperatur unter Argon 2 Stunden lang stehen, nach welcher Zeit man feststellt, dass das Ausgangsprodukt verschwunden ist (DSC, $CH_2Cl_2$/Aceton, 3:2). Dann wird das Gemisch oxydiert, indem man ungefähr 30 Minuten lang Luft durchperlt, und dann in wässrige 0,5 n-Salzsäure (100 ml) gegossen. Nach üblicher Extraktion mit Essigester erhält man 2 g Rückstand, und Filtrieren über Kieselsäure liefert 1,6 g (80%) reines Produkt, dessen Kenndaten den oben beschriebenen entsprechen.

BEISPIEL 17

1,2,3,4,6,11-Hexahydro-1(R),3(S),5,12-tetrahydroxy-3-C-hydroxymethyl-6,11-naphthacendion (20) und dessen 1(S)-Isomer der Formel (19):

Zu einer Lösung des gemäss Beispiel 15 erhaltenen Produkts (1,2 g, 3,3 mMol) in einem Gemisch aus THF (60 ml) und Methanol (60 ml) gibt man unter Argon bei -10°C eine wässrige, Natriumhydroxyd (450 mg) und Natriumhydrosulfit (730 mg, 4,2 mMol) enthaltende Lösung (30 ml). Dann lässt man die Temperatur wieder auf 0°C ansteigen und rührt ungefähr 1 1/2 Stunden lang bei dieser Temperatur. Das Reaktionsgemisch wird dann wie oben (Beispiel 16) behandelt. Man erhält 840 mg (d.h. 70%) Rohprodukt, welches das gewünschte Produkt als Gemisch mit dem Ausgangsstoff enthält. Diese wurden nach Schutz des primären Hydroxyls in der Form des p-Methoxytriphenylmethyl- oder p-Anisylderivats getrennt. Obwohl schwieriger, lässt sich die Trennung auch

direkt durchführen, wobei man in der Reihenfolge ansteigender Polarität das 1(R),3(S)-Isomer und danach das 1(S),3(S)-Isomer gewinnt.

<u>1(R),3(S)-Isomer (20)</u>: Schmelzpunkt 210-212 $^{O}$C; $\alpha_D^{20}$ - 108$^{O}$ (c 0,05, THF);

IR $\nu_{max.}^{CHCl_3}$: 3250 (OH), 1620 und 1590 $cm^{-1}$ (Chinon mit Wasserstoffbrücken);

NMR (Pyridin-$d_5$, 400 MHz):

8,39 (2H, m) und 7,75 (2H, m) (System AA' und BB', 4H aromatisch)

5,86 (1H, t, J = J' = 6, H-1)

4,28 (1H, d) und 4,21 (1H, d) (System AB, J = 10,5, $CH_2$-13)

3,60 (1H, d) und 3,39 (1H, d verbreitert) (System AB, J = 18, $CH_2$-4)

2,77 (1H, m, J = 13,5, J' = 6, J" = 1, H-2e) und 2,67 (1H, dd, J = 13,5, J' = 6,5, H-2a)

Massenspektrum (E.I.): m/z 356 ($M^+$, 50%), 338 (M - 18, 100), 320 (M - 18 - 18, 65), 307 (M - 18 - 31, 85) und 279 (65)

Analyse: Berechnet für $C_{19}H_{16}O_7$ (356,32) %:

C 64,04, H 4,53, O 31,43

<u>1(S),3(S)-Isomer (19)</u>: Schmelzpunkt 230 $^{O}$C; $\alpha_D^{20}$ + 95$^{O}$ (c 0,05, THF);

IR $\nu_{max.}^{CHCl_3}$ gleich wie das vorangehende Produkt.

NMR (Pyridin-$d_5$, 400 MHz):

8,37 (2H, m) und 7,73 (2H, m) (System AA' und BB', 4H aromatisch).

5,63 (1H, s verbreitert, H-1).

4,11 (1H, d) und 4,06 (1H, d) (System AB, J = 11, $CH_2$-13).

3,67 (1H, dd, J = 18,5, J' = 2, H-4a) und 3,24 (1H, d, J = 18,5, H-4e).

2,72 (1H, m, J = 14, J' = J" = 2, H-2e) und 2,30 (1H, dd, J = 14, J' = 4,5, H-2a).

Massenspektrum (E.I.): m/z 356 = ($M^{+\cdot}$, 56%), 338 (M - 18, 100), 320 (M - 18 - 18, 68), 307

(M - 18 - 31, 88), 279 (69).

Hohe Auflösung bei m/z 356: Berechnet 356,08977;
Gefunden: 356,335.

BEISPIEL 18

1,2,3,4,6,11-Hexahydro-1(R),3(S),5,12-tetrahydroxy-3-C-hydroxymethyl-13-p-methoxytriphenylmethyl-6,11-naphthacendion und dessen (cis)-1(S)-Isomer.

Eine Lösung des Gemischs der beiden Isomeren aus Beispiel 17 (1 g, 2,8 mMol) in Pyridin versetzt man bei -10°C mit p-Methoxytriphenylmethylchlorid (3,3 g, 11,3 mMol). Nach beendeter Zugabe lässt man das Reaktionsgemisch 24 Stunden bei Raumtemperatur stehen. Nach Verdünnen mit Methanol (5 ml) und 15 Minuten Rühren verdünnt man mit Wasser und extrahiert mit Dichlormethan. Bei Behandlung der organischen Phase in üblicher Weise verbleibt nach dem Eindampfen ein Rückstand (1,1 g), der über Kieselsäure (150 g, Dichlormethan/Methanol 999:1, V/V) chromatographiert wird. Nacheinander gewinnt man dabei das trans-1(R),3(S)-Isomer 20b und dann das cis-1(S),3(S)-Isomer 19b.

trans-Isomer: NMR ($CDCl_3$, 400 MHz):

8,28 (2H, m) und 7,80 (2H, m) (System AA' und BB', 4H aromatisch).
7,52 (2H, d, J = 9) und 6,82 (2H, d, J = 8) (System AA' und BB', p-Anisyl).
7,37-7,18 (10H, m, aromatisch)
5,33 (1H, dd, J = 8, J' = 6,5, H-1).
4,06 (1H, m, OH).
3,74 (3H, s, Anisyl-OMe).
3,27 (2H, s, $CH_2$-13).
2,92 (1H, d) und 2,81 (1H, d) (System AB, J = 18, $CH_2$-4).
2,49 (1H, m, J = 14, J' = 6,5, J" = 3,5) und 1,83 (1H, m) ($CH_2$-2).

cis-Isomer: NMR ($CDCl_3$, 400 MHz):

8,25 (2H, m) und 7,79 (2H, m) (System AA' und BB', 4H aromatisch)
7,50 (2H, d, J = 9) und 6,87 (2H, d, J = 9) (System

AA' und BB', p-Anisyl).

7,47-7,15 (10H, m, aromatisch).

5,16 (1H, dd, J = 4, J' = 1, H-1).

3,79 (3H, s, Anisyl-OMe).

3,24 (2H, s, $CH_2$-13).

2,70 (1H, d) und 2,32 (1H, d) (System AB, J = 18, $CH_2$-4).

2,32-1,82 (2H, m, $CH_2$-2).

BEISPIEL 19

1,4-Dihydroxy-2-(3'-desoxy-2'-C-hydroxymethyl-2',2"-O-isopropyliden-D-ribityl)-anthrachinon der Formel (21):

Eine Lösung des gemäss Beispiel 13 erhaltenen Produkts (4 g, 8,3 mMol) in Eisessig (200 ml) versetzt man mit Wasser (45 ml). Nach 1 Stunde Rühren bei Raumtemperatur zeigt DSC (Hexan/Aceton 2:1), dass das Ausgangsprodukt fast verschwunden ist, und es erscheint nur ein einziger, stärker polarer Flecken, der dem Monoisopropylidenderivat ohne Spuren des Tetrols (Beispiel 14) entspricht. Die Reaktion wird dann durch vorsichtigen Zusatz einer gesättigten Natriumbicarbonatlösung abgebrochen. Extraktion mit Essigester liefert ungefähr 4 g Rohprodukt. Dieses wird über Kieselsäure H 60 chromatographiert (500 ml-Fraktionen mit Dichlormethan/Methanol 99:1 als Laufmittel). Das Ausgangsprodukt (0,5 g) erhält man dabei in den ersten Fraktionen. Die nachfolgenden Fraktionen liefern das Monoisopropylidenderivat (3,5 g, 88%). Eine Analysenprobe erhält man durch Umkristallisierung aus Aceton: Schmelzpunkt 156-166$^o$; $(\alpha)_D^{20}$ + 60$^o$ (c 1, Chloroform);

IR $\nu_{max.}^{CHCl_3}$: 3620 (OH), 1625 und 1590 $cm^{-1}$ (Chinon mit Wasserstoffbrücken)

NMR ($CDCl_3$, 270 MHz):

13,42 (1H, s) und 12,69 (1H, s) (2 phenolische OH mit Wasserstoffbrücken).

8.17 (2H, m) und 7,74 (2H, m) (System AA' und BB', 4H aromatisch).

7,31 (1H, s, H-3); 4,08 (1H, m, H-4')

4,00 (2H, s, $CH_2$-5')
3,58 (1H, d) und 3,43 (1H, d) (System AB, J = 11, $CH_2$-2").
3,16 (1H, d) und 2,99 (1H, d) (System AB, J = 14, $CH_2$-1').
1,76 (1H, m, H-3').
1,41 (3H, s) und 1,33 (3H, s) (2 $CMe_2$).

Massenspektrum (DCI/$NH_3$): m/z 429 (M + 1, 100%), 371 (M + 1 - 58, 16%), 353 (M + 1 - 58 - 18, 17%).

Analyse: Berechnet für $C_{23}H_{24}O_8$ (428,42):
C 64,48, H 5,65, O 29,88
Gefunden: C 64,59, H 5,77, O 29.97

BEISPIEL 20

1,4-Dihydroxy-2-(2'(S)-hydroxy-2'-C-hydroxymethyl-2',2"-O-isopropyliden-butanal-4')-anthrachinon der Formel (22):

Man behandelt die Verbindung aus Beispiel 19 unter denselben Bedingungen wie in Beispiel 15 beschrieben (1,4 g 21, 100 ml Methanol, 50 ml THF und 1,4 g $NaJO_4$, d.h. 6,5 mMol, in 15 ml Wasser und 16 ml Eisessig). Dabei erhält man quantitativ 1,3 g des gewünschten Produkts, das laut DSC (Hexan/Aceton 2:1) einheitlich ist. Eine Analysenprobe erhält man durch Umkristallisieren aus Hexan: Schmelzpunkt 186-189°; $(\alpha)_D^{20}$ + 51 ° (c 0,07, Chloroform);
IR $\nu_{max.}^{CHCl_3}$: 1720 (CHO), 1625 und 1590 $cm^{-1}$ (Chinon mit Wasserstoffbrücken).

NMR ($CDCl_3$, 250 MHz):
13,24 (1H, s) und 12,64 (1H, s) (2 phenolische OH mit Wasserstoffbrücken).
9,65 (1H, s, CHO).
8,30 (2H, m) und 7,72 (2H, m) (System AA' und BB', 4H aromatisch).
7,18 (1H, s, H-3).
4,10 (1H, d) und 3,97 (1H, d) (System AB, J = 11, $CH_2$-2").
3,21 (1H, d) und 2,99 (1H, d) (System AB, J = 14,

$CH_2$-1').

2,85 (1H, d) und 2,58 (1H, d) System AB, J = 16, $CH_2$-3').

1,40 (6H, s, $CMe_2$).

Massenspektrum ($DCI/NH_3$): m/z 414 (M + 1 + $NH_3$, 5%); 397 (M + 1, 100%), 381 (M - 15, 20%) 356 (2%), 339 (M + 1 - Aceton), 321 (10%).

Analyse: Berechnet für $C_{22}H_{20}O_7$ (396,38):

C 66,66, H 5,09, O 28,25

Gefunden: C 66,80, H 5,12, O 28,33

BEISPIEL 21

1,2,3,4,6,11-Hexahydro-1(S),3(S),5,12-tetrahydroxy-3-C hydroxymethyl-3,13-O-isopropyliden-6,11-naphthacendion der Formel (23):

1. Bei -10°C und dann 0°C: das Aldehydderivat wird unter den zur Herstellung von 19 schon beschriebenen Bedingungen behandelt (300 mg Aldehyd, 40 ml THF/Methanol (1:1), 120 mg Natriumhydroxyd, 140 mg Natriumhydrosulfit und 10 ml Wasser). Dabei gewinnt man nach 2 Stunden bei 0°C 270 mg Rohprodukt. Dieses wird über Kieselsäure (Laufmittel: Toluol/Aceton 98:2) chromatographiert. Dabei erhält man nacheinander 65 g 14 und danach 60 mg 23 (Gesamtausbeute: 50%).

2. Bei -40°C: wird dieselbe Umsetzung bei -40°C durchgeführt, so findet man, dass laut DSC (Toluol/Aceton, 98:2) nur 23 vorliegt. So ergeben 300 mg 22 300 mg kristallisiertes Rohprodukt, das aus Aceton/Methanolgemisch umkristallisiert wird. Dabei erhält man reines 23 als erstes Kristallisat (165 mg, 55%): Schmelzpunkt 270-274°, $(\alpha)_D^{20}$ + 96° (c 1, Chloroform);

IR $\nu_{max.}^{CHCl_3}$: 3500 (OH), 1625 und 1590 $cm^{-1}$ (Chinon mit Wasserstoffbrücken)

NMR ($CDCl_3$, 270 MHz):

13,32 (1H, s) und 13,12 (1H, s) (2 phenolische OH mit Wasserstoffbrücken).

8,17 (2H, m) und 7,72 (2H, m) (System AA' und BB') (4H aromatisch).
5,14 (1H, m, J = 10, J' = 5, J" = 2, H-1).
4,08 (1H, d, J = 10, OH verschwindet nach Deuterierung).
3,96 (2H, s, $CH_2$-13).
3,21 (1H, dd, J = 18,5, J' = 1,5, H-4e) und 2,70 (1H, d, J = 18,5, H-4a).
2,43 (1H, m, J = 14, J' = 2, J" = 1,5, H-8e) und 1,98 (1H, dd, J = 14, J' = 5, H-8a).
1,48 (3H, s) und 1,43 (3H, s) (2 $CMe_2$).

Massenspektrum (DCI/$NH_3$): m/z (M + 1, 45%), 396 ($M^+$, 100%), 381 (M - 15, 20%), 320 (20%).

Analyse: Berechnet für $C_{22}H_{20}O_7$ (396,38) %:
C 66,66, H 5,09, O 28,25
Gefunden: C 66,72, H 5,18, O 27,98.

BEISPIEL 22

1-O-(3',4'-Di-O-acetyl-2',6'-didesoxy-α-L-lyxo-hexapyranosyl)-1,2,3,4,6,11-hexahydro-1(S),3(S),5,12-tetrahydroxy-3-C-hydroxymethyl-3,13-O-isopropyliden-6,11-naphthacendion der Formel 25:

Zu einer Lösung von 23 (75 mg, 0,19 mMol) in trockenem Dichlormethan (25 ml) gibt man gelbes Quecksilberoxyd (600 mg), Mercuribromid (210 mg) und Molekularsieb 4A (900 mg). Nach 1 Stunde Rühren bei Raumtemperatur versetzt man mit dem Chlorzucker 24 (125 mg, 0,50 mMol) in Lösung in 10 ml $CH_2Cl_2$. Nach 12 Stunden Rühren filtriert man das Reaktionsgemisch und extrahiert das Filtrat mit Dichlormethan mit nachfolgendem Waschen mit gesättigter Natriumbicarbonatlösung und dann mit Wasser. Nach Trocknen und Abdampfen des Lösungsmittels bei vermindertem Druck erhält man einen Rückstand von ungefähr 200 mg Gewicht. Chromatographie über Kieselsäure (Laufmittel: Toluol/Aceton, 95:5) liefert 100 mg reines kristallisiertes 25 (87% Ausbeute). Zur Analyse wird eine Probe aus Hexan/Aceton umkristallisert: Schmelzpunkt 199-200°; $(\alpha)_D^{20}$ + 105° (c 0,04, Chloroform);
IR $\nu_{max.}^{CHCl_3}$: 1740, 1225 und 1075 (Ester-CO), 1625 und 1590

(Chinon mit Wasserstoffbrücken).

NMR ($CDCl_3$, 270 MHz):

13,58 (1H, s) und 13,30 (1H, s) (2 phenolische OH mit Wasserstoffbrücken).

8,28 (2H, m) und 7,80 (2H, m) (System AA' und BB', 4H aromatisch).

5,58 (1H, d, J = 3, J' <1, H-4').

5,24 (1H, m, H-3').

5,20 (1H, s verbreitert, Halbwerts-J = 5, H-1).

5,03 (1H, t, J = 5, J' = 4, H-1').

4,40 (1H, q, J = 6,5, J' <1, H-5').

3,95 (1H, d) und 3,85 (1H, d) (System AB, J = 9, $CH_2$-13).

3,27 (1H, d) und 2,80 (1H, d) (System AB, J = 19, $CH_2$-4).

1,90-2,35 (4H, m, $CH_2$-2 und 2').

2,16 (3H, s) und 1,89 (3H, s) (2 OAc).

1,48 (6H, s, $CMe_2$).

1,21 (3H, d, J = 6,5, $CH_3$-6').

Massenspektrum DCI/$NH_3$: m/z 628 (M + 18, 10%), 440 (10), 396 (30), 379 (15), 250 (30), 183 (60), 155 (100%).

Analyse: Berechnet für $C_{32}H_{34}O_{12}$ (610,59) %:
C 62,94, H 5,61, O 31,44

Gefunden: C 62,78, H 5,68, O 31,58.

BEISPIEL 23

1-O-(3',4'-Di-O-acetyl-2',6'-didesoxy-α-L-lyxo-hexapyranosyl)-1,2,3,4,6,11-hexahydro-1(S),3(S),5,12-tetrahydroxy-3-C-hydroxymethyl-6,11-naphthacendion der Formel 26:

Man rührt das Glykosid 25 (40 mg) 4 Stunden lang bei Raumtemperatur in 0,2n methanolischer Salzsäure (30 ml). Dann giesst man das Reaktionsgemisch in gesättigte Natriumbicarbonatlösung (15-20 ml) und extrahiert in üblicher Weise mit Dichlormethan. Nach Abdampfen des Lösungsmittels bei vermindertem Druck gewinnt man 40 mg Rohprodukt. Dieses wird über Kieselsäure chromatographiert (Laufmittel: $CH_2Cl_2$/

0156172

Methanol 98:2). Nacheinander gewinnt man dabei 22 mg Ausgangsprodukt <u>25</u> und 18 mg <u>26</u>. Behandelt man die 22 mg <u>25</u> in der gleichen Weise, so erhält man ein zweites Kristallisat <u>26</u> (8 mg), d.h. insgesamt 26 mg (≅ 65%). Eine Probe wird zur Analyse aus Hexan/Aceton 1:1 umkristallisiert: Schmelzpunkt 207-209°; $(\alpha)_D^{20}$ + 91° (c 0,05, Chloroform);
IR $\nu_{max.}^{CHCl_3}$: 3500 (OH), 1740, 1200-1250, 1020 (Ester), 1625 und 1590 $cm^{-1}$ (Chinon mit Wasserstoffbrücken)

NMR ($CDCl_3$, 270 MHz):
13,60 (1H, s) und 13,30 (1H, s) (2 phenolische OH mit Wasserstoffbrücken).
8,28 (2H, m) und 7,80 (2H, m) (System AA' und BB', 4H aromatisch).
5,68 (1H, d, J = 3, H-4').
5,32 (1H, t verbreitert, J = 4, J' = 1, H-1').
5,26 (1H, s verbreitert, Halbwerts-J 5, H-1).
5,08 (1H, m, H-3').
4,34 (1H, q, J = 6, J'<1, H-5').
3,76 (1H, d) und 3,54 (1H, dd) (System ABX, J = 10, J' = 8, $CH_2$-13).
3,28 (1H, d) und 2,62 (1H, d) (System AB, J = 19, $CH_2$-4).
2,35-1,90 (4H, m, $CH_2$-2' und 2).
2,20 (3H, s) und 1,98 (3H, s) (2 OAc).
1,23 (3H, d, J = 6,5, $CH_3$-6').

Massenspektrum: (DCI/$NH_3$): m/z 588 (M + 18, 10%), 356 (15), 155 (100).

Wie aus dem obigen ersichtlich ist, beschränkt sich die Erfindung keineswegs auf solche Ausführungs- und Anwendungsformen, wie oben näher beschrieben, sondern umfasst sämtliche Varianten, die einem Fachmann naheliegen könnten, ohne vom Umfang oder dem Gedanken der Erfindung abzuweichen.

Patentansprüche:

1. Neue Reihe Anthracyclinone, dadurch gekennzeichnet, dass diese 3,13-O-Isopropyliden-anthracyclinone darstellen und der nachfolgenden allgemeinen Formel II entsprechen:

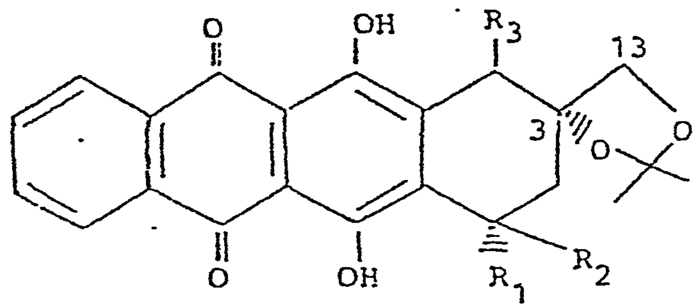

II.

worin $R_1$, $R_2$ und $R_3$ für eine OH-Gruppe oder ein Wasserstoffatom stehen.

2. Verfahren zur Herstellung von Anthracyclinonen nach Anspruch 1, gekennzeichnet durch die folgenden Stufen:

1) Umwandlung einer Saccharinsäure der nachfolgenden allgemeinen Formel III:

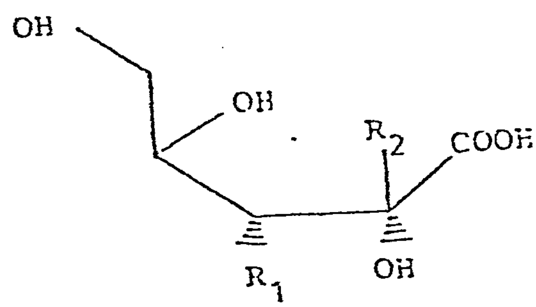

III.

worin $R_1$ = H oder OH und
$R_2$ = $CH_3$ oder $CH_2OH$,
in das Lacton der allgemeinen Formel III.bis

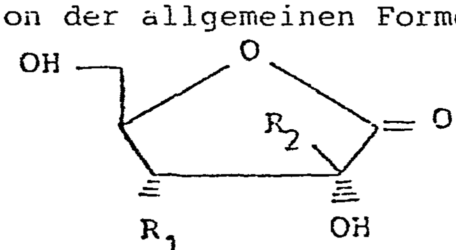

III.bis

worin $R_1$ und $R_2$ die oben angegebenen Bedeutungen haben, dann Ueberführung des Lactons in einen Aldit und anschliessende Oxydation einer endständigen (primären) Alkoholfunktion zur Aldehydfunktion, wobei diese Umwandlung gemäss Formelbild IV erfolgt, wenn man von 3-Desoxy-2-hydroxymethylpentonsäure ausgeht, bzw. Formelbild V,

wenn man von 2-Methyl-D-ribopentonsäure ausgeht.

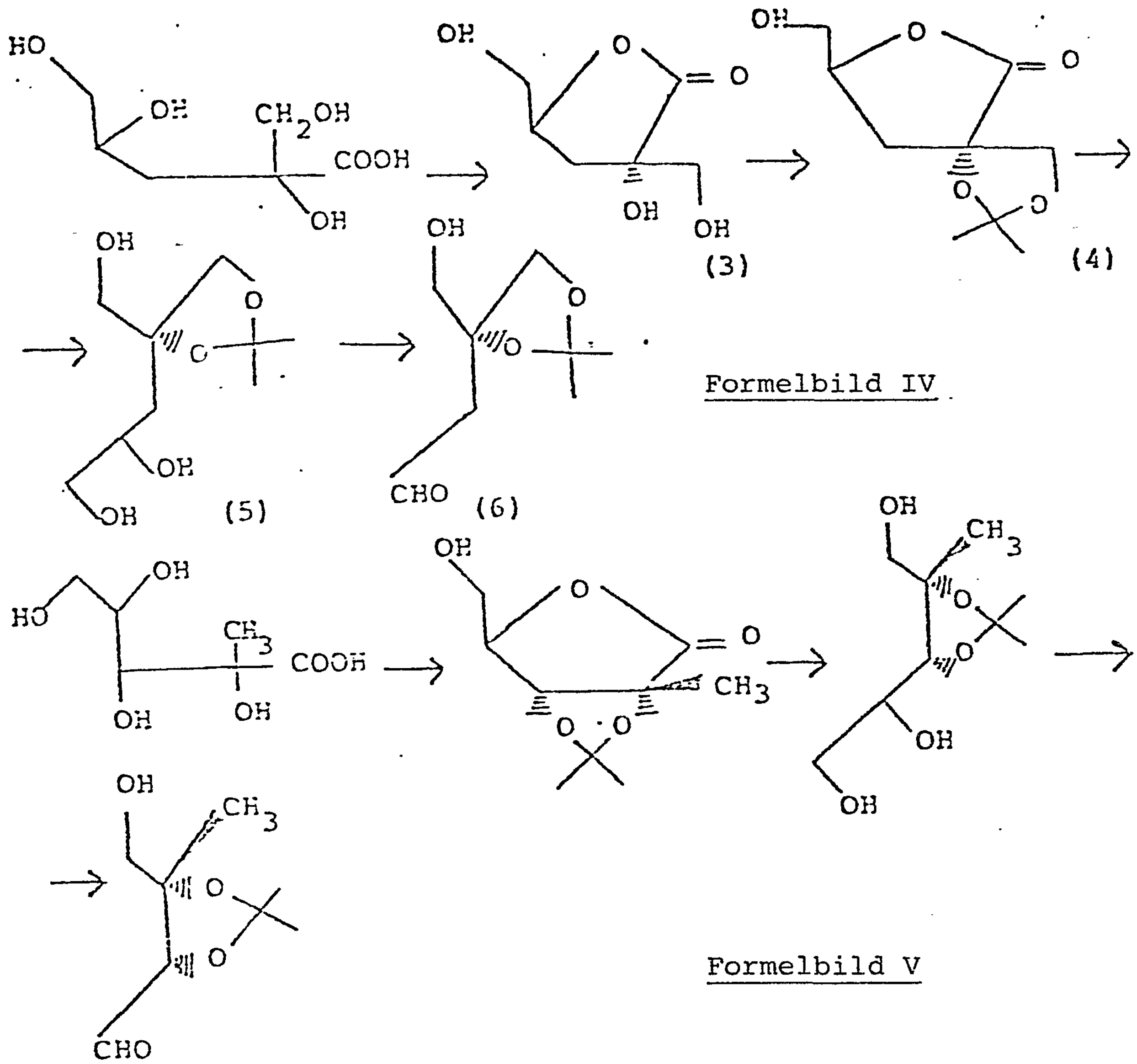

(3) (4) (5) (6)

Formelbild IV

Formelbild V

2) Kondensationsreaktion des Aldehyds mit Leukochinizarin und nachfolgende Oxydation, Cyclisierung, Hydrogenolyse und schliesslich Hydrolyse gemäss nachfolgendem Formelbild VI:

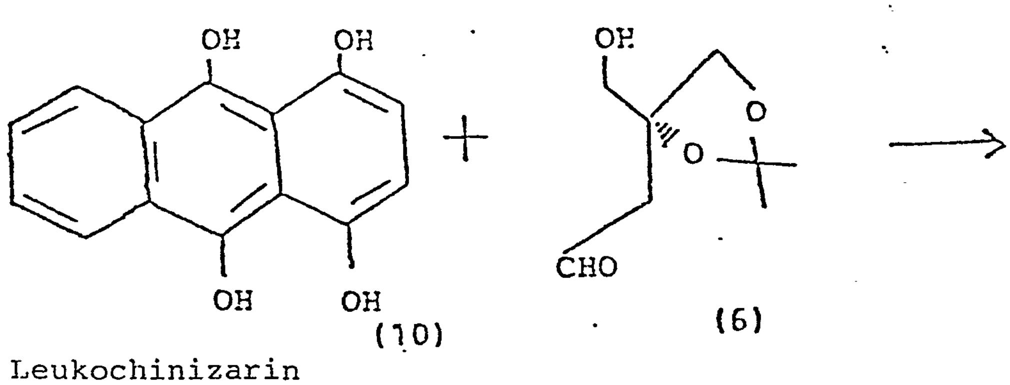

(10) (6)

Leukochinizarin

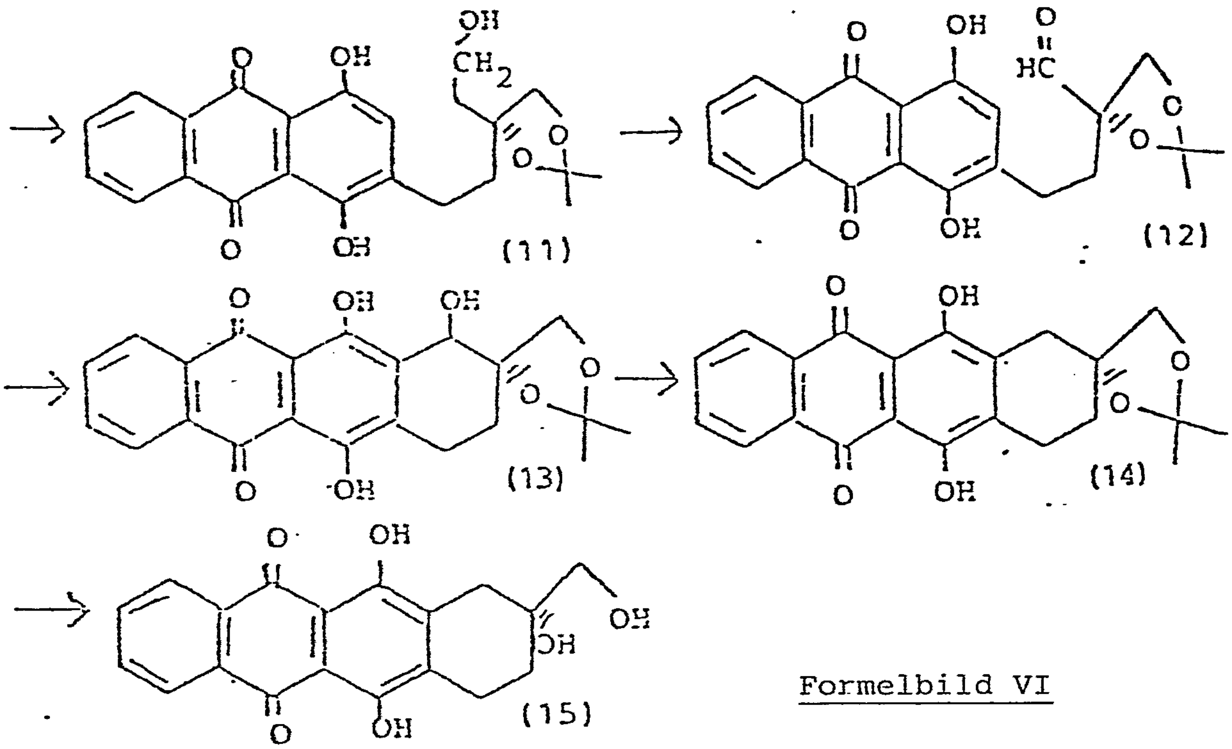

(11) (12) (13) (14) (15)

Formelbild VI

3. Verfahren zur Herstellung von Anthracyclinonen nach Anspruch 1, dadurch gekennzeichnet, dass man das in der ersten Stufe nach Anspruch 2 erhaltene Isopropylidenlactonderivat (4) in das entsprechende Amidderivat umwandelt, davon ein Diisopropylidenderivat bildet und dieses in den Aldehyd überführt, gemäss nachfolgendem Formelbild VII:

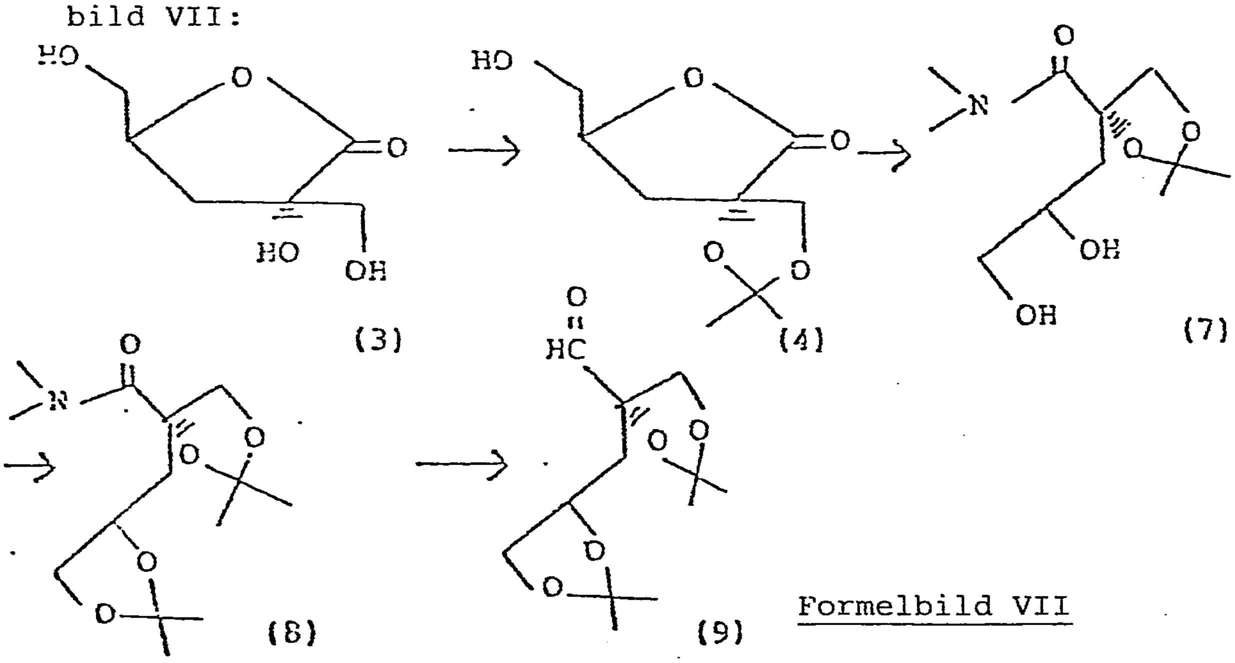

(3) (4) (7) (8) (9)

Formelbild VII

4. Verfahren nach Anspruch 3, dadurch gekennzeichnet, dass man den nach Formelbild VII erhaltenen Aldehyd (9) gemäss nachfolgendem Formelbild VIII mit Leukochinizarin zum

gewünschten cis-Anthracyclinon-derivat kondensiert:

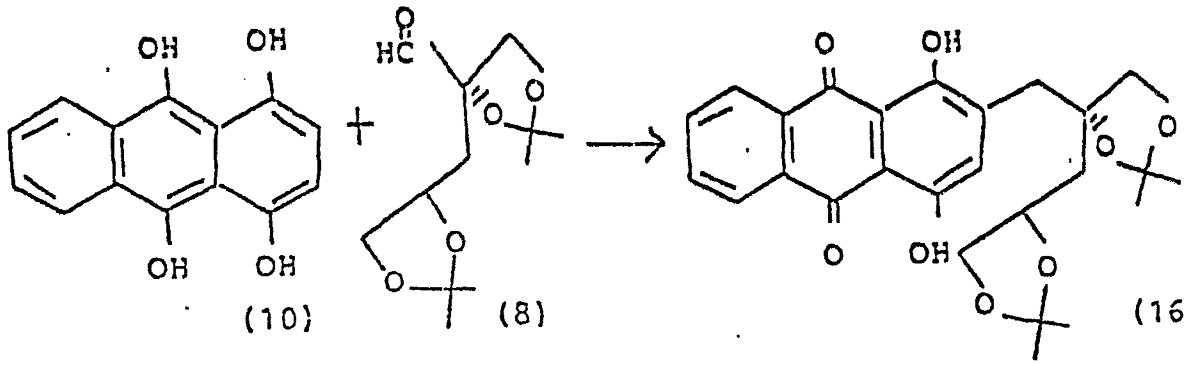

(10) (8) (16)

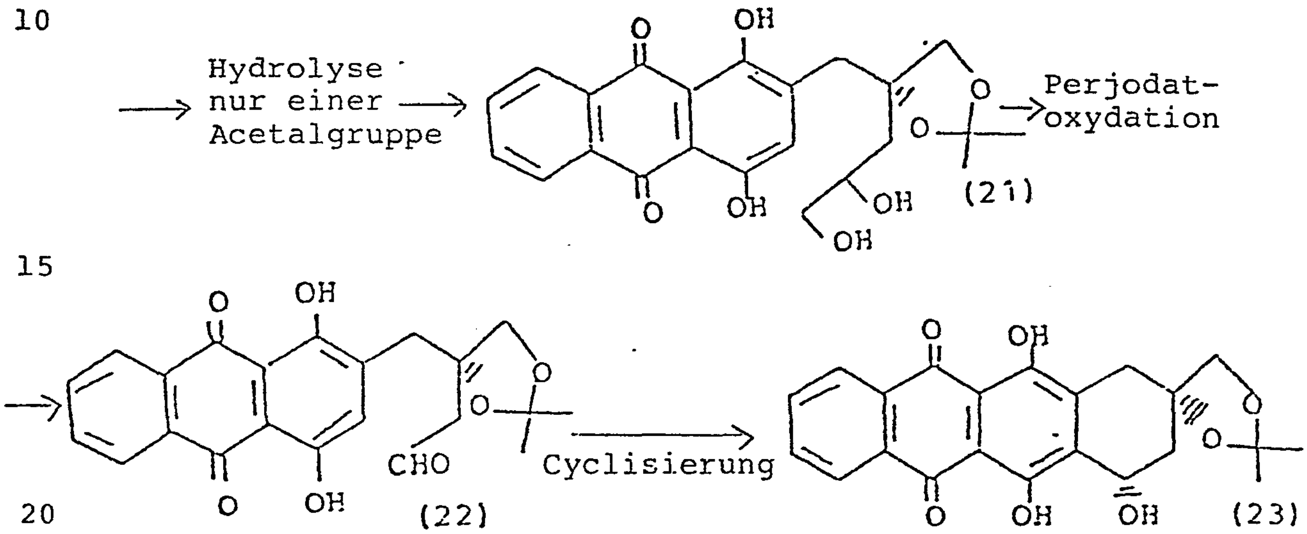


(21) (22) (23)

Formelbild VIII

5. Verfahren nach Anspruch 3, dadurch gekennzeichnet, dass zunächst das Diacetal (16) hydrolysiert und dann oxydiert und schliesslich durch eine Aldolisierungsreaktion zu einem Gemisch aus cis- und trans-Anthracyclinon cyclisiert wird, welches man durch Chromatographie trennt und gegebenenfalls durch Umsetzung mit α,α-Dimethoxypropan in saurem Medium in Isopropylidenderivate überführt, gemäss nachfolgendem Formelbild X:

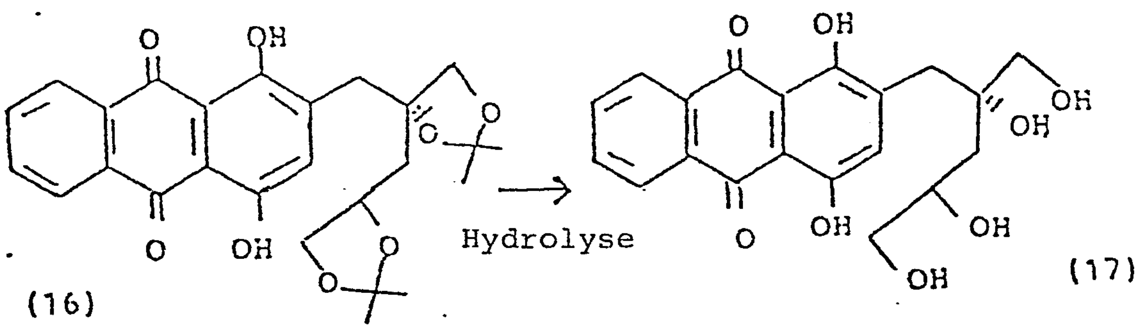


(16) (17)

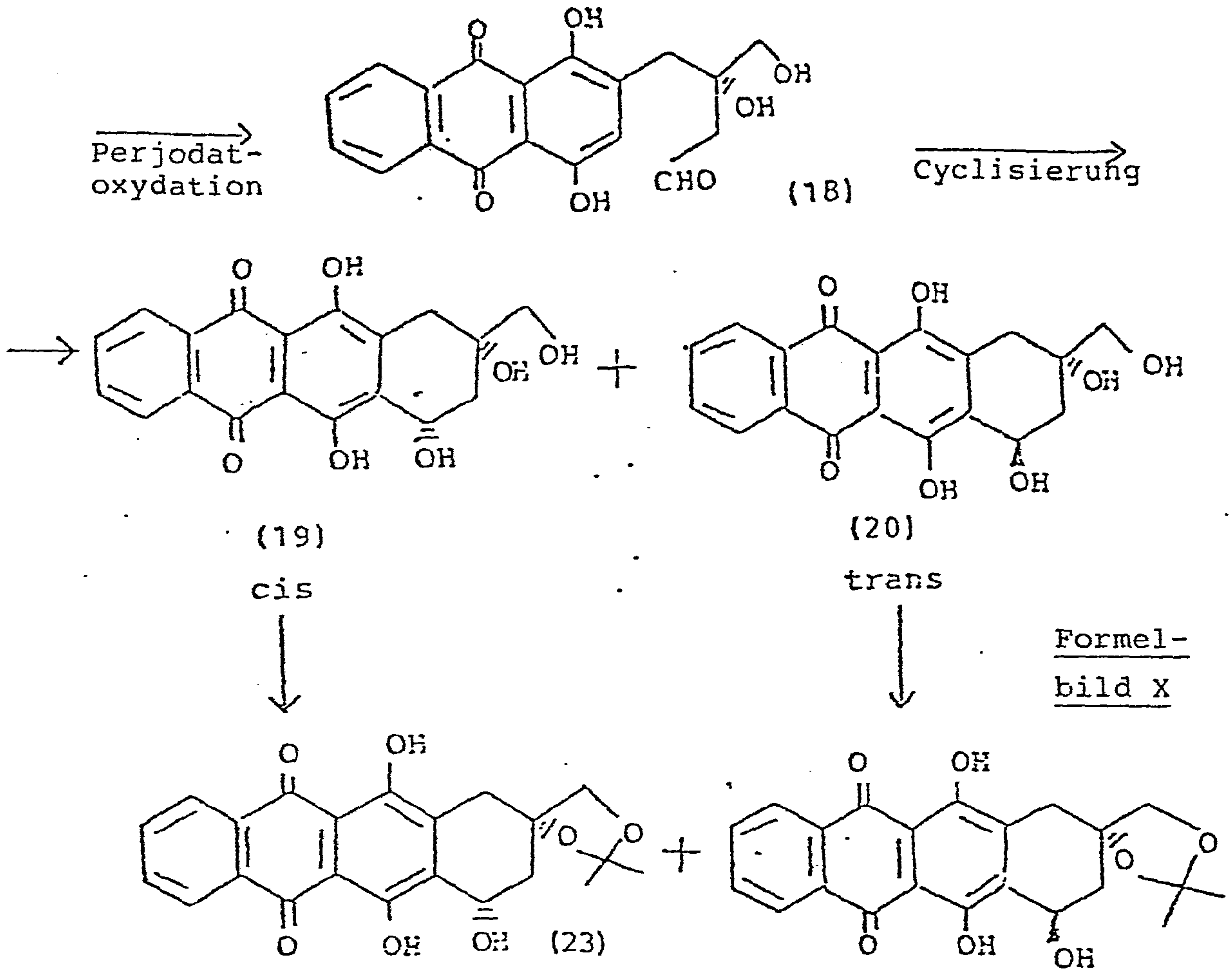


(18)

(19) cis

(20) trans

Formelbild X

(23)

6. Verfahren nach Anspruch 5, dadurch gekennzeichnet, dass die chromatographische Trennung der cis- und trans-Derivate nach Schutz der Alkoholfunktion der Hydroxymethylgruppe mit einer p-Methoxytriphenylmethylgruppe XI erfolgt.

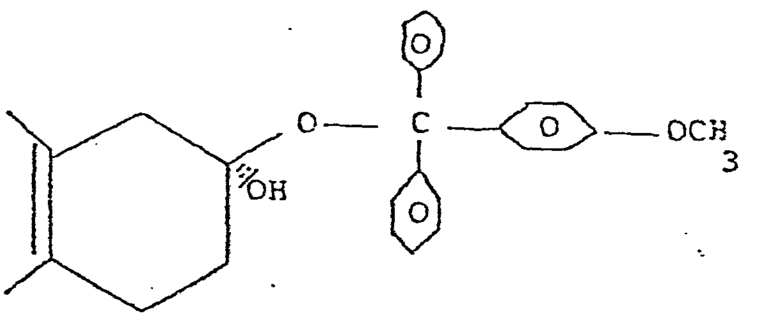

XI

7. Verfahren zur Umwandlung der Isopropylidenanthracyclinone in Anthracycline durch Glykosidierung mittels 3,4-Di-O-acetyl-2,6-didesoxy-α-L-lyxohexosechlorid (24) nach der Koenigs-Knorr-reaktion, gemäss nachfolgendem Formelbild XII:

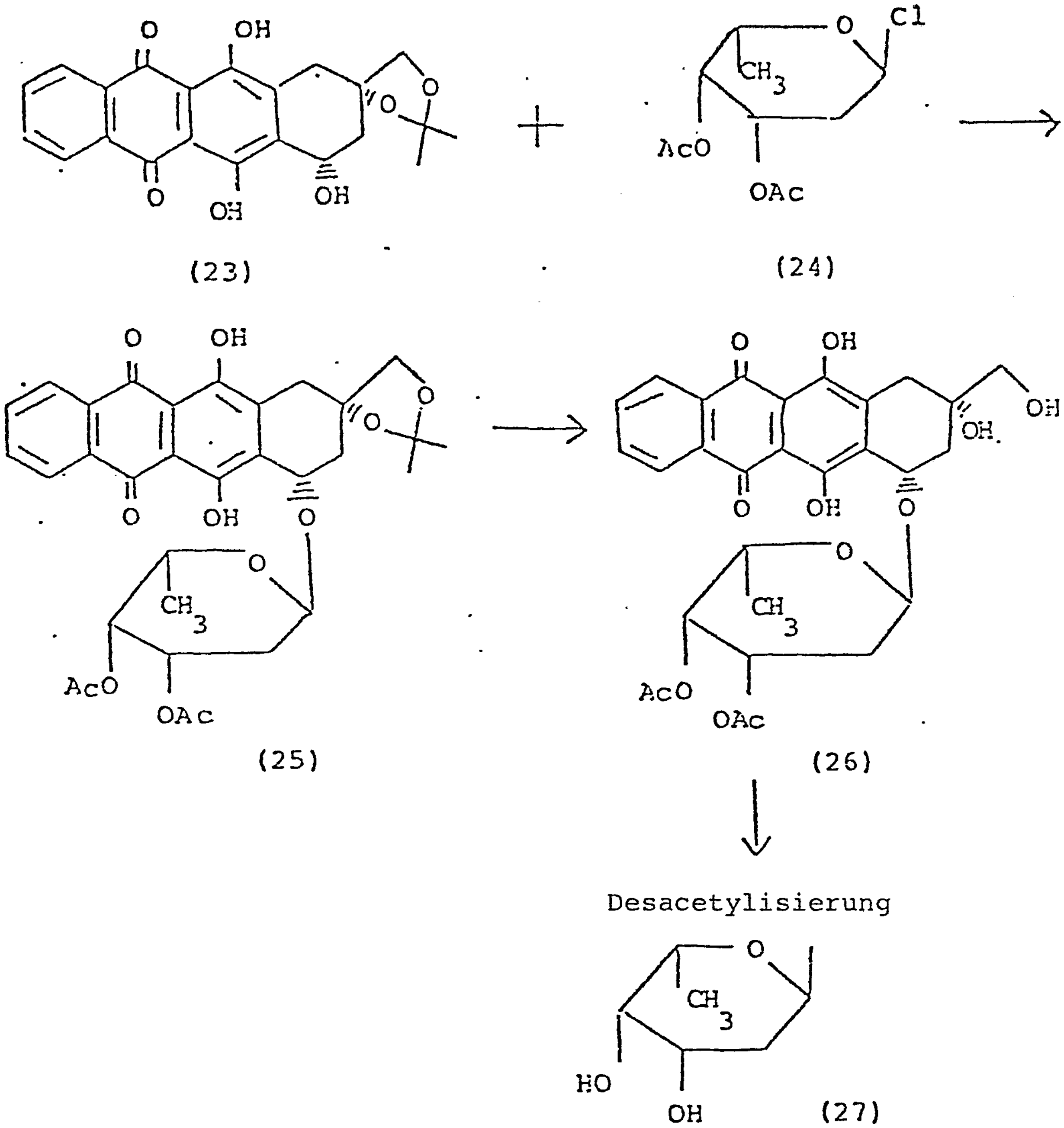


(23) (24)

(25) (26)

(27)

8. Anthracyclinon, bestehend aus 1,2,3,4,6,11-Hexahydro-3(R),4(R und S),5,12-tetrahydroxy-3-C-hydroxymethyl-3,13-O-isopropyliden-6,11-naphthacendion der nachfolgenden Formel (13):

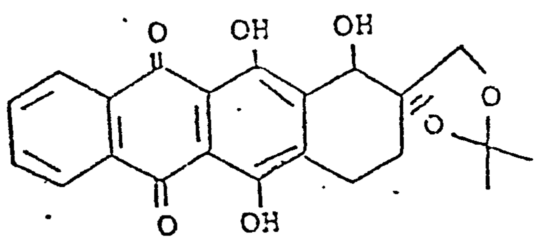

(13)

9. Anthracyclinon, bestehend aus 1,2,3,4,6,11-Hexahydro-3(R),5,12-trihydroxy-3-C-hydroxymethyl-3,13-O-isopropyliden-6,11-naphthacendion der nachfolgenden Formel (14):

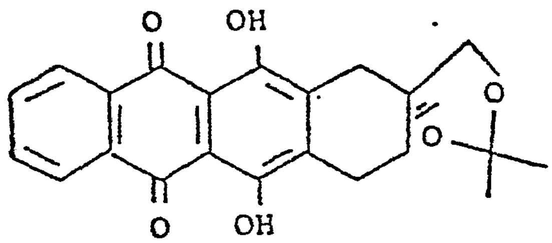

(14)

10. Anthracyclinon, bestehend aus 1,2,3,4,6,11-Hexahydro-3(R),5,12-trihydroxy-3-C-hydroxymethyl-6,11-naphthacendion der nachfolgenden Formel (15):

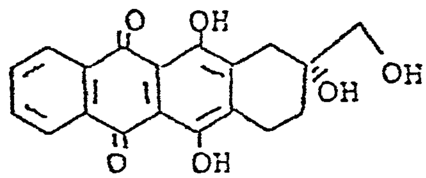

(15)

11. Anthracyclinon bestehend aus 1,2,3,4,6,11-Hexahydro-1(S),3(S),5,12-tetrahydroxy-3-C-hydroxymethyl-6,11-naphthacendion der nachfolgenden Formel (19):

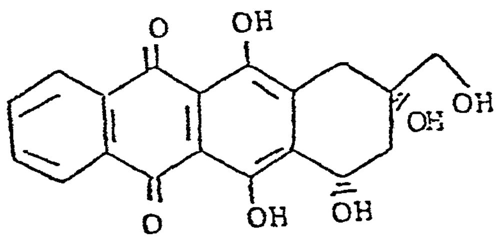

(19)

12. Anthracyclinon, bestehend aus 1,2,3,4,6,11-Hexahydro-1(R),3(S),5,12-tetrahydroxy-3-C-hydroxymethyl-6,11-naphthacendion der nachfolgenden Formel (20):

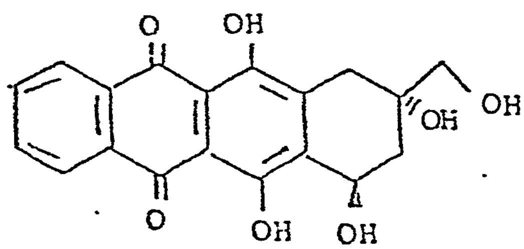

(20)

13. Anthracyclinon, bestehend aus 1,2,3,4,6,11-Hexahydro-1(S),3(S),5,12-tetrahydroxy-3-C-hydroxymethyl-3,13-O-isopropyliden-6,11-naphthacendion der nachfolgenden Formel (23):

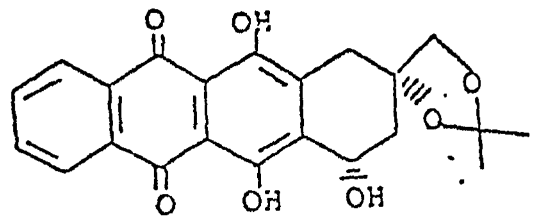

(23)

14. Anthracyclin, bestehend aus 1-O-(3',4'-Di-O-acetyl-2',6'-didesoxy-α-L-lyxo-hexopyranosyl)-1,2,3,4,6,11-hexahydro-1(S),3(S),5,12-tetrahydroxy-3-C-hydroxymethyl-3,13-O-isopropyliden-6,11-naphthacendion der nachfolgenden Formel (25):

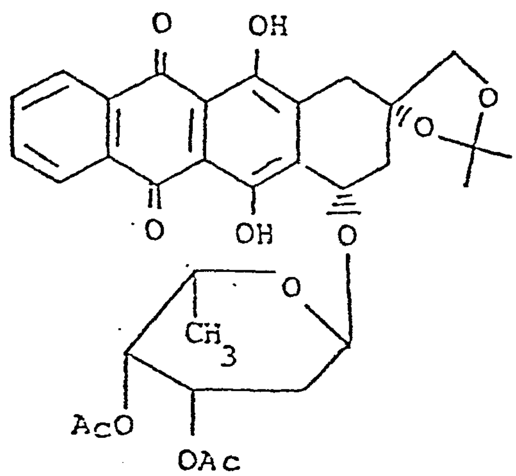

(25)

15. Anthracyclin, bestehend aus 1-O-(3',4'-Di-O-acetyl-2',6'-didesoxy-α-L-lyxo-hexopyranosyl)-1,2,3,4,6,11-hexahydro-1(S),3(S),5,12-tetrahydroxy-3-C-hydroxymethyl-6,11-naphthacendion der nachfolgenden Formel (26):

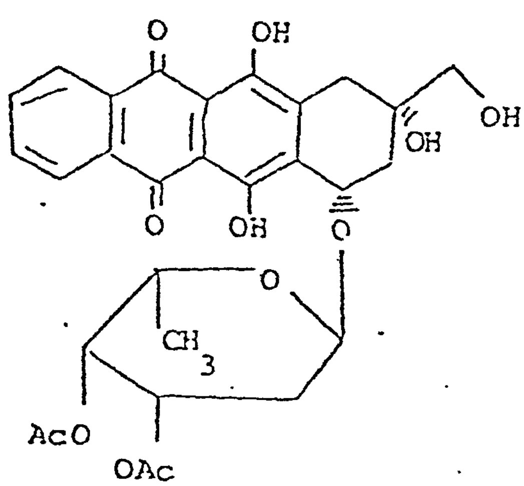

(26)

16. Anthracyclin, bestehend aus 1-O-(2',6'-Didesoxy-α-L-lyxo-hexopyranosyl)-1,2,3,4,6,11-hexahydro-1(S),3(S),5,12-tetrahydroxy-3-C-hydroxymethyl-6,11-naphthacendion

der nachfolgenden Formel (27):

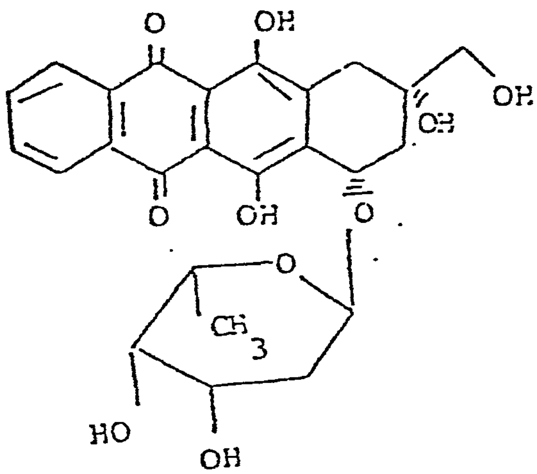

(27)